# EUROPEAN PATENT SPECIFICATION

(11) **EP 1 423 363 B1**
(45) Date of publication and mention of the grant of the patent: **19.04.2006**
(21) Application number: 02745739.9
(22) Date of filing: 15.07.2002
(51) Int. Cl.: C07D 207/32, C07D 263/32, C07D 413/12, C07D 403/12, C07D 401/12

(54) **ORAL ANTIDIABETIC AGENTS**
ORALE ANTIDIABETISCHE WIRKSTOFFE
AGENTS ANTIDIABETIQUES ORAUX

(30) Priority: 29.08.2001 US 315728 P
(43) Date of publication of application: 02.06.2004
(60) Divisional application: 05104581.3
(73) Proprietor: Warner-Lambert Company LLC, Morris Plains, New Jersey 07950 (US)
(72) Inventor: BIGGE, Christopher F., Pfizer Global Res. & Dev., Ann Arbor, MI4810 5 (US); BRIDGES, Alexander J., Pfizer Global Res. & Dev., Ann Arbor, MI 48105 (US); CASIMIRO-GARCIA, A., Pfizer Global Res.& Dev., Ann Arbor,r, MI 48105 (US); FAKHOURY, Stephen A. Pfizer Global Res. & Dev., Ann Arbor, MI 48105 (US); LEE, Helen T. Pfizer Global Res. & Dev., Ann Arbor, MI 48105 (US); REED, Jessica E. Pfizer Global Res. & Dev., Ann Arbor, MI 48105 (US); SCHAUM, Robert P. Pfizer Global Res. & Dev., Ann Arbor, MI 48105 (US); SCHLOSSER, Kevin M. Pfizer Global Res. & Dev., Ann Arbor, MI 48105 (US); SEXTON, Karen E. Pfizer Global Res. & Dev., Ann Arbor, MI 48105 (US); ZHOU, Hairong Pfizer Global Res. & Dev., AnnArbor, MI 4810 5 (US)
(74) Representative: Rutt, Jason Edward
(86) International application number: PCT/IB2002/002843
(87) International publication number: WO 2003/018553

(56) References cited:
- EP-A- 1 067 109
- WO-A-00/23425
- WO-A-00/66572
- WO-A-01/17994
- WO-A-99/08501
- US-B1- 6 274 608
- SHINKAI H. ET AL: 'Isoxazolidine-3,5-dione and Noncyclic 1,3-Dicarbonyl Compounds as Hypoglycemic Agents' JOURNAL OF MEDICINAL CHEMISTRY vol. 41, no. 11, 1998, AMERICAN CHEMICAL SOCIETY. WASHINGTON, US, pages 1927 - 1933, XP002176728

## Description

### FIELD OF THE INVENTION

The present invention relates to compounds that are useful as antidiabetic agents.

### BACKGROUND OF THE INVENTION

Type II diabetes, or non-insulin dependent diabetes (NIDDM) is a significant healthcare problem whose incidence is on the rise. Between 1990 and 1998, the prevalence of NIDDM in the United States increased by 33 percent, to about 13 million persons. An additional 5 million persons are presumed to have undiagnosed NIDDM, while another 14 million persons have impaired glucose tolerance. Direct medical costs associated with diabetes were $44 billion in 1997, due mainly to hyperglycemia-related diabetic complications, including diabetic angiopathy, atherosclerosis, diabetic nephropathy, diabetic neuropathy, and diabetic ocular complications such as retinopathy, cataract formation, and glaucoma.

NIDDM is one of a number of disease states associated with the phenomenon of insulin resistance. Insulin resistance is defined as the reduced sensitivity to the actions of insulin in the whole body or individual tissues, such as skeletal muscle tissue, myocardial tissue, fat tissue or liver tissue. Insulin resistance occurs in many individuals with or without diabetes mellitus. Insulin resistance syndrome (hereinafter IRS) refers to the cluster of manifestations that include insulin resistance; hyperinsulinemia; non insulin dependent diabetes mellitus (NIDDM); arterial hypertension; central (visceral) obesity; and dyslipidemia.

The primary goal of IRS therapy and thus diabetes therapy is to lower blood glucose levels so as to prevent acute and long-term disease complications. For some persons, modified diet and increased exercise may be a successful therapeutic option for achieving the goal of glucose control. When modified diet and increased exercise are not successful, drug therapy using oral antidiabetic agents is initiated.

To date, a number of oral antidiabetic agents have been developed. For instance, sulfonylureas are generally used to stimulate insuln. The biguanide metformin is generally used to improve insulin sensitivity and to decrease hepatic glucose output. Acarbose is used to limit postprandial hyperglycemia, Thiazolidine 2,4 diones are used to enhance insulin action.

New drug therapies for the treatment of NIDDM have focused in part on the discovery of new Peroxisome Proliferator Activation Recpetor (PPAR) agonists. PPARs are members of the nuclear receptor superfamily of transcription factors that includes steroid, thyroid, and vitamin D receptors. PPARs play a role in controlling expression of proteins that regulate lipid metabolism. There are three PPAR subtypes: PPAR α, PPAR δ, and PPAR γ.

Each PPAR receptor shows a different pattern of tissue expression, and differences in activation by structurally diverse compounds. PPAR γ, for instance, is expressed most abundantly in adipose tissue and at lower levels in skeletal muscle, heart, liver, intestine, kidney, vascular endothelial and smooth muscle cells as well as macrophages. Two isoforms of PPAR γ exist, identified as γ₁ and γ₂, respectively. PPAR γ mediates adipocyte signalling, lipid storage, and fat metabolism. Evidence gathered to date support the conclusion that PPAR γ is the primary, and perhaps the only, molecular target mediating the insulin sensitizing action of one class of antidiabetic agents, the thiazolidine 2,4 diones.

In a monotherapeutic or combination therapy context, new and established oral antidiabetic agents are still considered to have non-uniform and even limited effectiveness. The effectivieness of oral antidiabetic therapies may be limited, in part, because of poor or limited glycemic control, or poor patient compliance due to unacceptable side effects. These side effects include edema weight gain, or even more serious complications. For instance, hypoglycemia is observed in some patients taking sulfonylureas. Metformin, a substituted biguanide, can cause diarrhea and gastrointestinal discomfort. Finally, edema, weight gain, and in some cases, hepatoxicity, have been linked to the administration of some thiazolidine 2,4 dione antidiabetic agents. Combination therapy using two or more of the above agents is common, but generally only leads to incremental improvements in glycemic control.

As a result, there is a need for oral antidiabetic agents that can be used alone or in combination, and that do not give rise to side effects such as fluid retention, peripheral edema, weight gain, or more severe complications.

### SUMMARY OF THE INVENTION

These and other needs are met by the current invention which is a compound of formula (I) 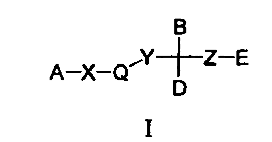 or a pharmaceutically acceptable salt thereof, wherein:
A is 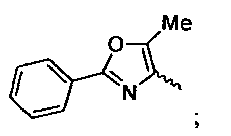
X is
   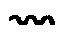 CH₂-CH₂-O 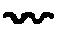 or 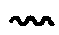 CH₂-CH₂-CH₂ 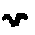 ,, wherein " 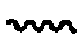 " indicates a point of attachment;
Q is 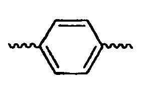
Y is CH₂;
Z is absent;
B is H;
D is 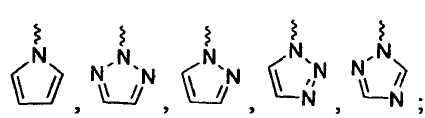 and;
E is CO₂H.

The invention also provides a compound which is:

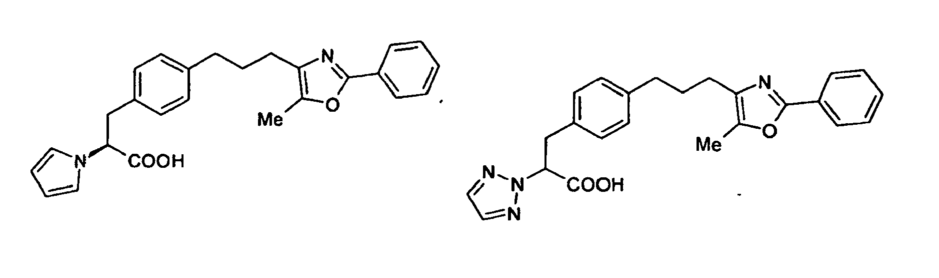

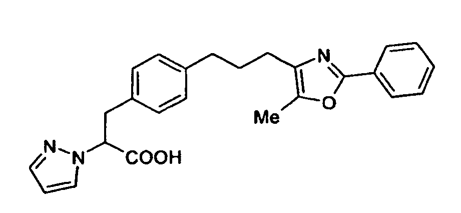

The invention also provides a pharmaceutical composition comprising a compound of formula I admixed with a carrier, diluent, or excipient.

The invention also provides a method of treating, preventing or controlling non-insulin dependent diabetes mellitus in a mammal comprising administering to the mammal in need thereof an effective amount of a compound of formula I.

The invention also provides a method of treating, preventing or controlling obesity in a mammal comprising administering to the mammal in need thereof an effective amount of a compound of formula I.

The invention also provides a method of reducing body weight in an obese mammal comprising administering to the mammal in need thereof an effective amount of a compound of formula I.

The invention also provides a method of treating, preventing or controlling hyperglycemia in a mammal comprising administering to the mammal in need thereof an effective amount of a compound of formula I.

The invention also provides a method of treating, preventing or controlling hyperlipidemia in a mammal comprising administering to the mammal in need thereof an effective amount of a compound of formula I.

The invention also provides a method of treating, preventing or controlling hypercholesteremia in a mammal comprising administering to the mammal in need thereof an effective amount of a compound of formula I.

The invention also provides a method of treating, preventing or controlling atherosclerosis in a mammal comprising administering to the mammal in need thereof an effective amount of a compound of formula I.

The invention also provides a method of treating, preventing or controlling hypertriglyceridemia in a mammal comprising administering to the mammal in need thereof an effective amount of a compound of formula I.

The invention also provides a method of treating, preventing or controlling hyperinsulinemia in a mammal comprising administering to the mammal in need thereof an effective amount of a compound of formula I.

The invention also provides a method of treating a patient suffering from abnormal insulin and/or evidence of glucose disorders associated with circulating glucocorticoids, growth hormone, catecholamines, glucagon, or parathyroid hormone, comprising administering to the patient a therapeutically effective amount of a compound of of formula I.

The invention also provides a method of treating insulin resistance syndrome in humans comprising administering to a patient in need of treatment a composition of formula I.

The invention also provides a method of modulating PPAR activity in a mammal, comprising administering to a mammal an effective amount of a PPAR modulator of formula I.

The invention also provides a method of lowering blood glucose in a mammal, comprising administering to a mammal an effective amount of a compound of formula I.

The invention also provides a method of modulating fat cell differentiation in a mammal, comprising administering to a mammal an effective amount of a compound of formula I.

### DETAILED DESCRIPTION OF THE INVENTION

The term "diabetes" refers to a metabolic disorder in which there is impaired glucose utilization inducing hyperglycemia. An overview of the pathogenesis and morphology of diabetes and its late complications, particularly NIDDM, is available to practitioners of the art, for instance, in Robins' Pathologic Basis of Disease (5^{th} Ed. pp. 910-922). Other metabolic disorders associated with impaired glucose utilization and insulin resistance include IRS, described previously. In addition to the major late-stage complications of NIDDM (diabetic angiopathy, atherosclerosis, diabetic nephropathy, diabetic neuropathy, and diabetic ocular complications such as retinopathy, cataract formation and glaucoma), many other conditions are linked to NIDDM, including dyslipidemia glucocortcoid induced insulin resistance, dyslipidemia, polycysitic ovarian syndrome, obesity, hyperglycemia, hyperlipidemia, hypercholerteremia, hypertriglyceridemia, hyperinsulinemia, and hypertension. Brief definitions of these conditions are available in any medical dictionary, for instance, Stedman's Medical Dictionary (Xth Ed.).

As used herein, the term "modulate" means to change (something such as an action or a process); to make it more suitable for its situation. Cambridge Dictionaries Online (http:// dictionary. cambridge. org/define. asp?key= modulate*2+0 *last visited* June 20, 2002).

It will be appreciated by those skilled in the art that compounds of the invention having a chiral center may exist in and be in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine activity or cytotoxicity using the standard tests described herein, or using other similar tests which are well known in the art.

Specific and preferred values listed below for radicals, substituents, and ranges, are for illustration only; they do not exclude other defined values or other values within defined ranges for the radicals and substituents

Referring to a compound of formula (I), a specific value for A is

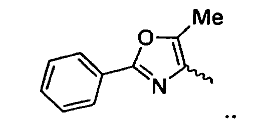

A specific value for X is 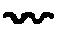 CH₂-CH₂-O 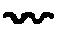 Wherein " 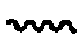 " indicates a point of attachment. Another specific value for X is 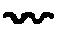 CH₂-CH₂-CH₂ 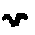 .

A specific value for Q is 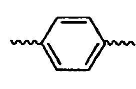 wherein " 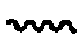 " indicates a point of attachment.

A specific value for Y is CH₂.

A specific value for Z is Z is absent.

A specific value for B is H.

A specific value for D is 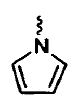 wherein " 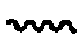 " indicates a point of attachment. Another specific value for D is 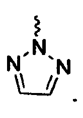 Another specific value for D is 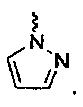 Another specific value for D is 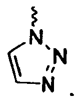 Still another specific value for D is

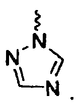

Another group of compounds of the invention are compounds of formula I wherein A is 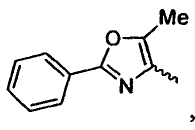 wherein " 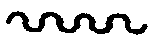 " indicates the point of attachment; X is 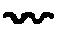 CH₂-CH₂-O- 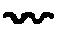 , or 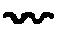 CH₂-CH₂-CH₂ 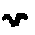 Q is 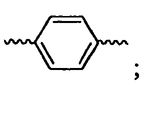 Y is CH₂; Z is absent; B is H; D is 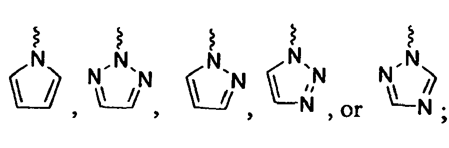 and E is CO₂H.

Another group of compounds of the invention are compounds of formula I wherein A is 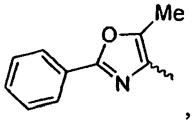 wherein " 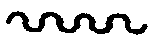 " indicates the point of attachment; X is 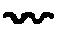 CH₂-CH₂-CH₂ 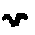 Q is 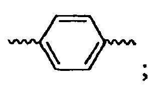 Y is CH₂; Z is absent; B is H;Dis 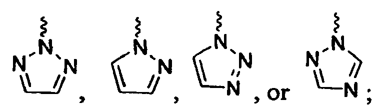 and E is CO₂H.

Another group of compounds of the invention are compounds of formula I wherein A is 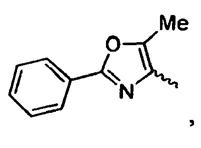 wherein " 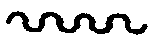 " indicates the point of attachment; X is 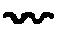 CH₂-CH₂-CH₂ 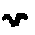 Q is 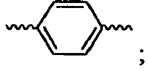 Y is CH₂; Z is absent; B is H; D is , 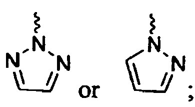 and E is CO₂H.

Another group of compounds of the invention are compounds of formula I wherein A is 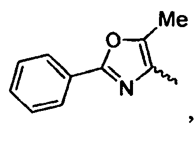 wherein " 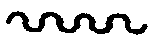 " indicates the point of attachment; X is 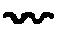 CH₂-CH₂-CH₂ 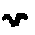 ; Q is 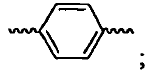 Y is CH₂; B is H; D is 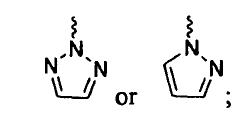 and E is CO₂H.

Processes and novel intermediates for preparing compounds of formula I are illustrated by the following procedures in which the meanings of the generic radicals are as given above unless otherwise qualified. Certain compounds of formula I are useful as intermediates for preparing other compounds of formula I.

It is also noted that compounds of formula I can be prepared using protecting groups. It is to be noted that the appropriate use and choice of protecting groups is well-known by one skilled in the art, and is not limited to the specific examples below. It is also to be understood that such groups not only serve to protect chemically reactive sites, but also to enhance solubility or otherwise change physical properties. A good general reference for protecting group preparation and deprotection is "Protecting Groups in Organic Synthesis" by T.W. Green and P.G. Wuts. A number of general reactions such as oxidations and reductions etc. are not shown in detail but can be done by methods understood by one skilled in the art. General transformations are well-reviewed in "Comprehensive Organic Transformation" by Richard Larock, and the series "Compendium of Organic Synthetic Methods" published by Wiley-Interscience. In general, the starting materials are obtained from commercial sources unless otherwise indicated.

It will be appreciated by those skilled in the art that compounds of the invention having one or more chiral centers may exist in and be isolated in optically active and racemic forms. Some compounds may exhibit polymorphism. It is to be understood that the present invention encompasses any racemic, optically-active, polymorphic, geometric, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine activity or cytotoxicity using the standard tests described herein, or using other similar tests which are well known in the art.

It will be further appreciated by the skilled artisan that the following schemes depic the synthesis of compounds wherein A, X, Q, Y, B, D, Z, or E are defined. It is to be understood however, that compounds of the invention other than those specifically disclosed can be prepared using the strategies depicted in the schemes.

Scheme 1 depicts a general approach to the preparation of compounds of Formula I wherein the pyrrolyl group is unsubstituted.

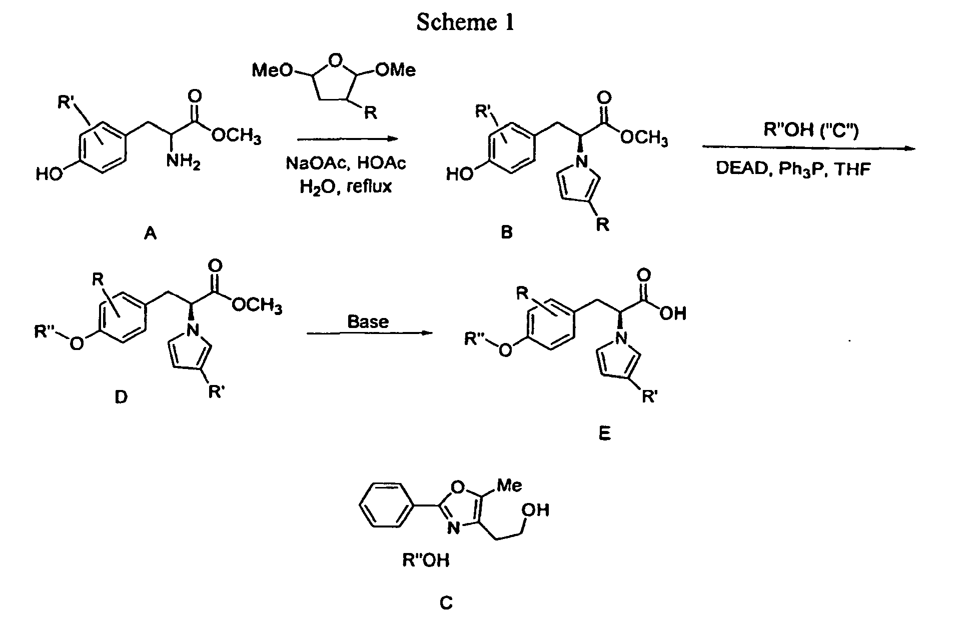

In Scheme 1, the pyrrolotyrosine ester B is first prepared by allowing tyrosine ester A to undergo reaction with 2,5 dimethoxy tetrahydrofuran in the presence of base. The methyl ester is depicted in Scheme 1, but it is possible that other esters may be used. Coupling of pyrrolotyrosine ester analogue with a compound bearing a primary or secondary alcohol under Mitsounobu-type or similar conditions provides pyrrolotyrosine methyl ester derivative D. Hydrolysis of pyrrolotyrosine methyl ester derivative D under basic conditions provides compound of the invention E.

Scheme 2 depicts the synthesis of compounds of the invention wherein X is ―CH―CH=CH― and D is heteroaryl.

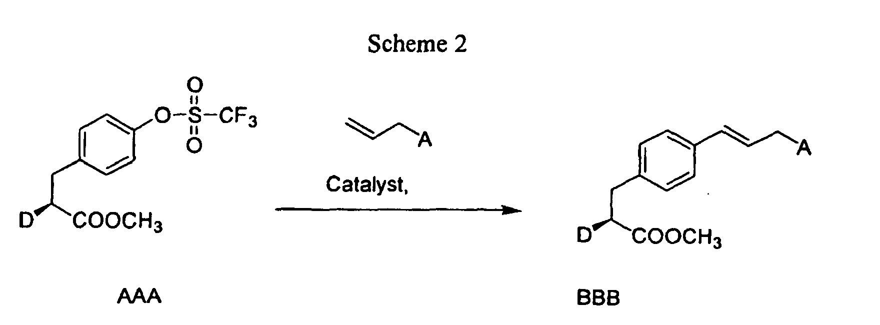

In Scheme 2, coupling of the triflate AAA with a vinyl compound in the presence of an organometallic catalyst provides vinyl ester BBB. Hydrolysis of the ester moiety in BBB as described above provides a compound of the invention wherein R is alkyl or substituted alkyl, aryl or substituted aryl, heteroaryl or substituted heteroaryl, heterocycloalkyl and substituted heterocycloalkyl, or heteroalkyl or substituted heteroalkyl.

Scheme 3 depicts the synthesis of additional compounds of the invention wherein X is ―C≡C-CH₂― and D is heteroaryl.

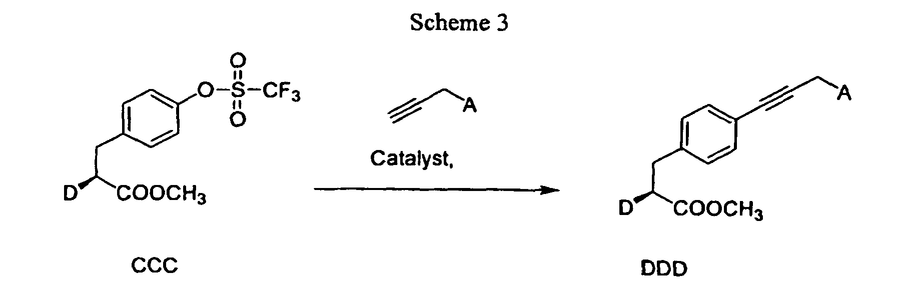

In Scheme 3, coupling of the triflate AAA with an alkynyl compound CCC in the presence of an organometallic catalyst provides alkynyl ester DDD. Hydrolysis of the ester moiety in DDD as described above provides a compound of the invention wherein R is alkyl or substituted alkyl, aryl or substituted aryl, heteroaryl or substituted heteroaryl, heterocycloalkyl and substituted heterocycloalkyl, or heteroalkyl or substituted heteroalkyl.

Scheme 4 depicts the synthesis of compounds of the invention wherein X is ―(CH₂)₃― and D is heteroaryl.

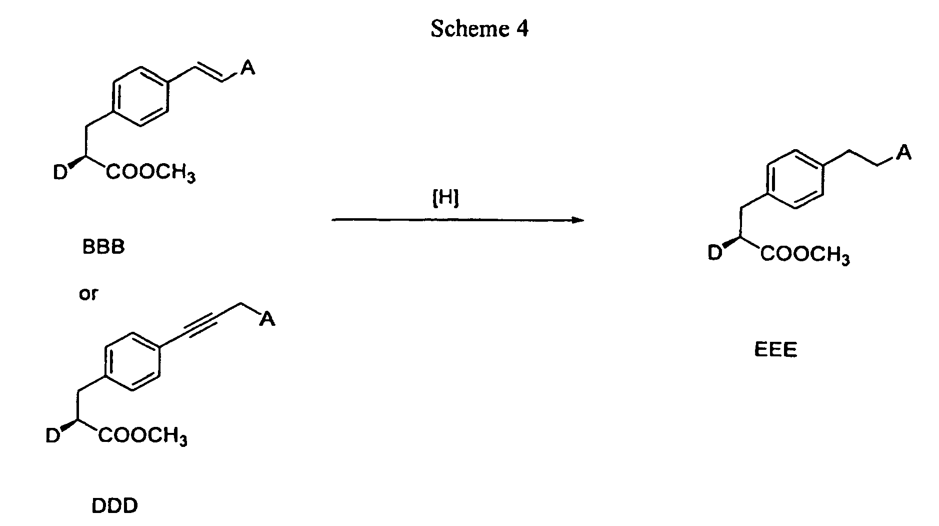

Scheme 4 indicates that either the vinyl compound BBB or the alkynyl compound DDD can be reduced under conditions known in the art to provide the saturated variant EEE.

Some of the compounds of Formula I are capable of further forming pharmaceutically acceptable acid-addition and/or base salts. All of these forms are within the scope of the present invention.

Pharmaceutically acceptable acid addition salts of the compounds of Formula I include salts derived from nontoxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogen phosphate, metaphosphate, pyrophosphate, acetate, trifluoroacetate, propionate, caprylate, isobutyrate, oxalate, malonate, succinates suberate, sebacate, fumarate, maleate, mandelate, benzoate, chlorobenzoate, methylbenzoate, dinitrobenzoate, phthalate, benzensoulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge S.M. et al., "Pharmaceutical Salts," *Journal of Pharmaceutical Science,* 1977;66:1-19).

The acid addition salt of basic compounds are prepared by contacting the free base form with a sufficient amount of the desired acid to produce the salt in the conventional manner.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge S.M., supra., 1977).

The base addition salts of acidic compounds are prepared by contacting the free acid form with a sufficient amount of the desired base to produce the salt in the conventional manner.

Certain of the compounds of the present invention can exist in unsolvated forms as well as solvated forms, including hydrated forms. In general, the solvated forms, including hydrated forms, are equivalent to unsolvated forms and are intended to be encompassed within the scope of the present invention.

Certain of the compounds of the present invention possess one or more chiral centers and each center may exist in the R(D) or S(L) configuration. The present invention includes all enantiomeric and epimeric forms, as well as the appropriate mixtures thereof.

The compounds of formula I can be formulated as pharmaceutical compositions and administered to a mammalian host, such as a human patient in a variety of forms adapted to the chosen route of administration, i.e., orally or parenterally, by intravenous, intramuscular, topical or subcutaneous routes.

Thus, the present compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1 % of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of formula I to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compounds of formula I can be determined by comparing their in vitro activity, and in vivo activity in animal models. Methods for the extrapolation of effective dosages in mice, and other animals, to humans are known to the art; for example, see U.S. Pat. No. 4,938,949.

Generally, the concentration of the compound(s) of formula I in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from about 0.005 to about 100 mg/kg, e.g., from about 0.1 to about 75 mg/kg of body weight per day, such as 0.03 to about 50 mg per kilogram body weight of the recipient per day, preferably in the range of 0.06 to 90 mg/kg/day, most preferably in the range of 0.15 to 60 mg/kg/day.

The compound may conveniently be administered in unit dosage form; for example, containing 0.05 to 1000 mg, conveniently 0.1 to 750 mg, most conveniently, 0.5 to 500 mg of active ingredient per unit dosage form.

Ideally, the active ingredient should be administered to achieve peak plasma concentrations of the active compound of from about 0.005 to about 75 µM, preferably, about 0.01 to 50 µM, most preferably, about 0.02 to about 30 µM. This may be achieved, for example, by the intravenous injection of a 0.0005 to 5% solution of the active ingredient, optionally in saline, or orally administered as a bolus containing about 0.01-1 mg of the active ingredient. Desirable blood levels may be maintained by continuous infusion to provide about 0.0001-5 mg/kg/hr or by intermittent infusions containing about 0.004-15 mg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The antidiabetes ability of a compound of the invention is demonstrated using pharmacological models that are well known to the art, for example, using models such as the tests described below.

### TEST A―3T3-L1 Adipocyte Differentiation Assay (ADPDIFF)

This assay is used to determine the potential of putative PPAR-γ ligands to induce fat cell differentiation. A quantitative method was established for determining the ability of potential PPAR-γ ligands to promote adipogenesis of 3T3-L1 preadipocytes. 3T3-L1 preadipocytes are plated onto 96 well plates (20,000 cells per well). Upon confluency, the compounds which were initially scored as positives from the PPAR-γ ligand displacement and PPAR-γ chimeric receptor transcription assays were added for 4 days with the final concentrations of 2. 5, 5.0 and 10 µM (n=3). Each plate contains positive controls (5 uM BRL 49653 and 5 uM Troglitazone) and a vehicle control (DMSO). Cells are replenished with FBS containing media on day 5 of post-drug treatment and incubated for additional 4-6 days. Cells are stained with BODIPY a fluorescent lipophilic stain to quantitate the lipid content of the cells. The assay is optimized for a 96-well plate format. Approximately after 10 days of post-drug treatment, cells are fixed in 3% formalin solution for 15 min followed by staining with BODIPY (80 µg/ml) for 20 min at room temperature. The plate is then put through the CYTOFLUOR instrument to measure the fluorescence of BODIPY (excitation=485/ 20; emission=530/ 25). A template is created in a spread sheet to calculate the average value of BODIPY measurements and reported as % of BRL 49653 at 5 µM.

### TEST B―ANTCV1 Antagonist Transcription Assay

The ANTCV1 transcription assay is an *in vitro* assay in which CV-1 cells, an African green monkey kidney cell line, are transfected with a luciferase reporter construct comprised of a fragment of the fatty acid binding protein (FATP) promoter located upstream of the TK TATA box. Transfection efficiency is controlled by co-transfectionof the reference plasmid CMV β-galactosidase. The DNAs are transiently transfected into the cells by electroporation and the cells are plated into 96-well dishes. Test compounds are diluted to final concentration of 25 µM in individual wells containing 300 nM BRL. Control wells include either the 0.5% DMSO vehicle control, the antagonist PD 0157724-0000 at 25 µM and 300nM BRL, or 300nM BRL alone. Cells are incubated with both the drugs for 48 hrs and then harvested. The lysates are measured for luciferase and β-galactosidase activities using the dual luciferase kit from Tropix on a EG&G Berthold MicroLumat LB96P luminometer. The fold activation of BRL 49653 in each assay must be above 4 in order for the assay to be considered valid.

Raw numbers are transferred to an Excel spreadsheet and luciferase/β-galactosidase ratios are determined for each compound. The percent BRL 49653 inhibition for each compound is calculated by dividing the luciferase/β-galactosidase ratio for each compound by the luciferase/β-galactosidase ratio of the DMSO vehicle control. This number is then plugged into the following equation: % BRL inhibition = (BRL fold activation - test compound fold activation & BRL / BRL fold activation -1) × 100.

### TEST C―CV-1 NATIVE RECEPTORS TRANSCRIPTION ASSAY (MKNRCV1)

The purpose of this assay is to identify ligands that activate endogenous nuclear receptors in CV-1 cells. Protocol: CV-1 cells are co-transfected with a luciferase reporter containing a fragment of the FATP promoter upstream the TK TATA box and a CMV beta-galactosidase plasmid. Transfected cells are incubated with test ligands for 48 hours. Cell lysates are harvested and the luciferase and beta-galactosidase activities are determined. Description: Luciferase and beta-galactosidase activities in the cell lysates are measured using an EG&G Berthold luminometer. These values are entered into and Excel worksheet which calculates the luciferase to beta-galactosidase ratios and expresses the data as percent activity of the reference compound, BRL 49653.

### TEST D―BLOOD GLUCOSE MEASURMENT (GLUCOSE Δ)

Glucose is obtained 4 hours post dose via tail vein stick (5µl whole blood) in awake, 4 hour fasted animals. Blood is drawn by capillary action into a glucose cuvette and read in a HemoCue Glucose Analyzer (Ryan Diagnostics). Blood is diluted 1:2 with saline if glucose levels are greater than 400mg/dl (meter high range) and results multiplied by two.

### TEST E―3T3-L1 TRANSIENT REPORTER ASSAY

This assay is used to determine the potential of putative PPAR-γ ligands to activate the promoter/enhancer of the murine aP2 gene. 3T3-L1 preadipocytes are cultured on collagen coated plates in DMEM containing 10% Calf serum for 48 hours post-confluence. The cells are then cultured for approximately 3 days in DMEM containing 10% Fetal Bovine Serum (FBS), 0.5-mM methyl isobutylxanthine, 0.25 µM dexamethasone, and 1 µg/ml of insulin. Cells are detached from the plates with Trypsin-EDTA and resuspended in Phosphate Buffered Saline (PBS).

The reporter construct used in this assay is comprised of the -5.4 Kb 5' flanking region of the murine aP2 gene inserted into the cloning site of the TKpGL3 luciferase vector. Transfection efficiency is controlled by co-transfection of the reference plasmid CMV-β-galactosidase. The reporter construct and the reference plasmid are transiently co-transfected into the cells by electroporation. The transfected cells are plated into collagen coated 96-well plates and cultured overnight. Cells are then incubated for 48 hours with the test compounds and then harvested. The lysates are measured for luciferase and β-galactosidase activities using the Dual-Light® luciferase kit from Tropix on a EG&G Berthold MicroLumat LB96P luminometer.

Data are summarized in Tables 1-3.

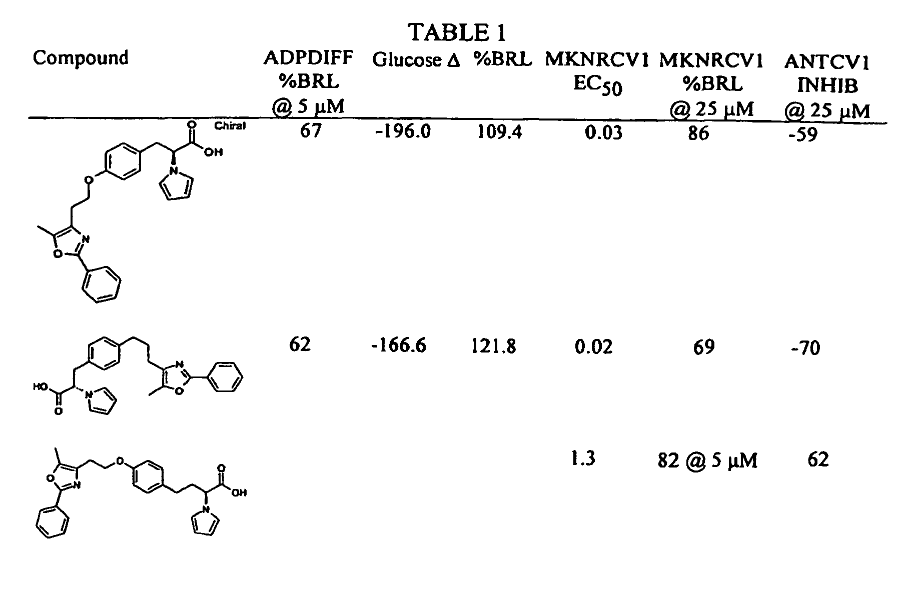

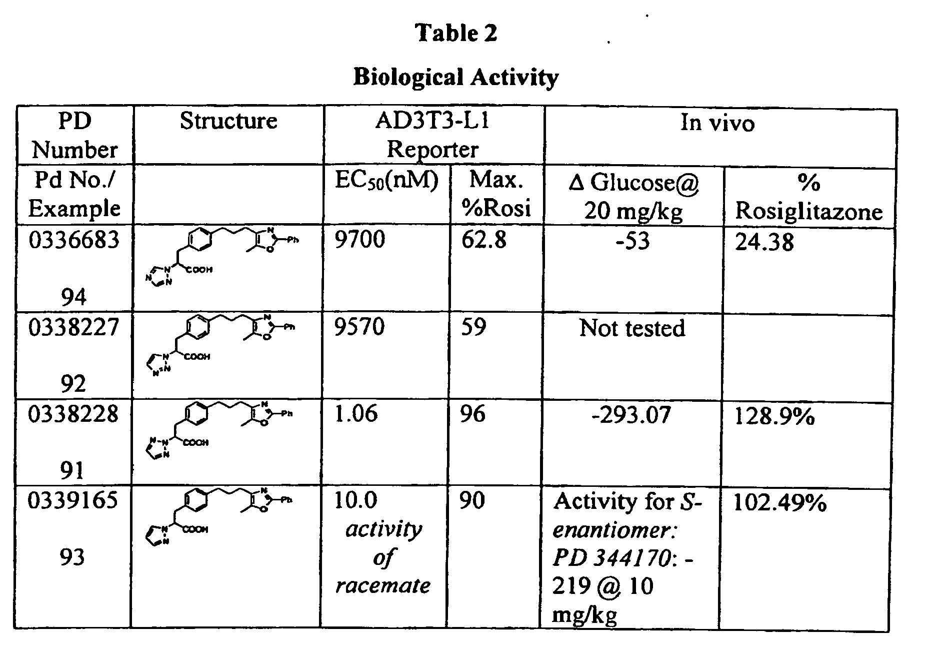

Referring to Table 2, PD 0338228 was evaluated for its selective activity against the three types of PPARs using HepG2 cells. In these screens, PD 338228 showed an EC₅₀ = 71.8 nM in the HepG2-hALPHA assay (PPAR alpha) and an EC50 = 92.5 nM in the HepG2-mGAMMA assay (PPAR gamma). It was inactive against PPAR beta. In the PPAR alpha assay, PD 3328228 at max. effect gives only 61 % of the max. activity of the reference agent (GW9578). The results obtained with Rosiglitazone and PD 326234 are included in the table shown below.

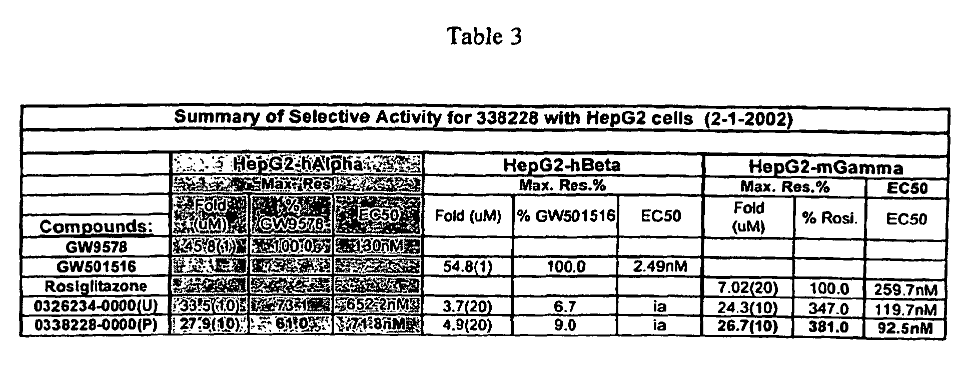

In the table, GW9578 refers to:

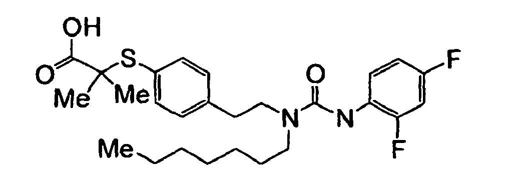

GW501516 refers to:

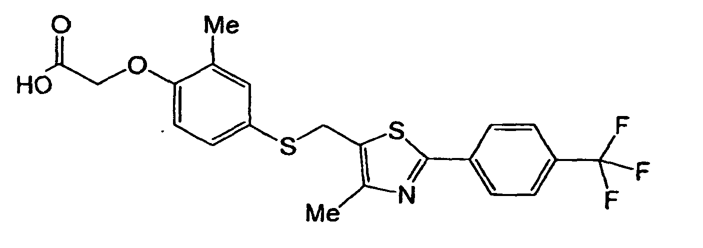

PD 338228 was identified as a potent, PPARgamma full agonist in the AD3T3-L1 reporter assay. In addition, this compound acts as a dual alpha/gamma agonist as demonstrated in the selectivity assays. It showed a partial agonist profile for PPARalpha. When evaluated in the Ob/Ob mice model for diabetes, PD 338228 was capable of completely normalizing plasma glucose levels at 20 mg/kg in 14 days. Its activity was comparable, or even better, than that of rosiglitazone. PD 338228 is a potential agent for the treatment of type II diabetes and dislipedemias, as that condition is described, for instance, in the Merck Manual (1992).

In general, compounds of the invention may induce fat cell differentiation as described in test A. Results from test E, in particular, demonstrate that the compounds of the invention may lower glucose levels in mice.

Because compounds of the invention induce adipocyte differentiation and lower blood glucose levels, they may be useful in treating disorders associated with insulin resistance. Such disorders include: NIDDM, diabetic angiopathy, atherosclerosis, diabetic nephropathy, diabetic neuropathy, and diabetic ocular complications such as retinopathy, cataract formation and glaucoma, as well as glucocortcoid induced insulin resistance, dyslipidemia, polycysitic ovarian syndrome, obesity, hyperglycemia, hyperlipidemia, hypercholerteremia, hypertriglyceridemia, hyperinsulinemia, and hypertension.

Accordingly, the invention also includes a method for treating a disease in a human or other mammal in need thereof in which insulin resistance has been implicated and glucose lowering is desired, comprising administering to said human or mammal an effective amount of a compound of formula I or a pharmaceutically acceptable salt thereof. The invention also includes a method for treating or preventing insulin resistance in a mammal comprising administering to said mammal an effective amount of a compound of formula I or a pharmaceutically acceptable salt thereof. Additionally, compounds of the invention can be used in vitro or in vivo as pharmacological and biochemical tools to assist in the discovery and evaluation of other glucose lowering agents and PPAR γ agonists.

### EXAMPLES

### Example 1

### (S)-3-{4-[2-(5-Methyl-2-phenyl-oxazol-4-yl)-ethoxy]-phenyl}-2-pyrrol-1-yl-propionic acid (3).

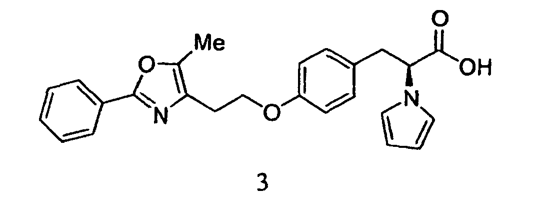

**General Procedure for Ester Hydrolysis (General Procedure A).** (S)-3-{4-[2-(5-Methyl-2-phenyl-oxazol-4-yl)-ethoxy]-phenyl}-2-pyrrol-1-yl-propionic acid methyl ester (400 mg, 0.930 mmol) was dissolved in THF (10 mL) and H₂O (10 mL). LiOH monohydrate (45 mg, 1.07 mmol) was added in one portion and the suspension stirred for 1 hour. The reaction was diluted with EtOAc (20 mL) and H₂O (20 mL) and organic layer separated. After acidification of the aqueous layer to pH of approximately 2 with aq.HCl and extraction with EtOAc (2x20 mL), the organic layers were combined and washed with H₂O (10 mL), brine (10 mL), and dried over MgSO₄. Removal of solvent *in vacuo* gave 3 as white crystals (320 mg, 0.769 mmol). Recrystalized from EtOAc/Hex as fine white needles in 51% yield. M.P. = 155-56 °C. C₂₅H₂₄N₂O₄ Mass Calc. = 416.475; M+1(obs) =417.2. ¹H NMR (400MHz) CDCl₃ δ: 7.91 (2H, m), 7.38 (3H, m), 6.90 (2H, d, *J*=8.5 Hz), 6.71 (2H, d, *J*=8.5 Hz), 6.69 (2H, t, *J*=2.0, 2.1 Hz), 6.12 (2H, t, *J= 1*.9, 1.9 Hz), 4.69 (1H, dd, *J* =7.08, 8.03 Hz), 4.16 (2H, t, 6.5, 6.5 Hz), 3.34 (1H, dd, *J*=6.6, 6.6 Hz), 3.17 (1H, dd, *J*=8.3, 8.3 Hz), 2.93 (2H, t, *J*=6.6, 6.6 Hz), 2.32 (3H, s). CHN (theoretical=C=72.10, H=5.81, N=6.73, CHN obs.; C=72.09, H=5.58, N=6.63.

(S)-3-{4-[2-(5-Methyl-2-phenyl-oxazol-4-yl)-ethoxy]-phenyl}-2-pyrrol-1-yl-propionic acid methyl ester (2) was prepared in the following manner.

### (a) (S)-3-{4-[2-(S-Methyl-2-phenyl-oxazol-4-yl)-ethoxy]-phenyl}-2-pyrrol-1-yl-propionic acid methyl ester (2).

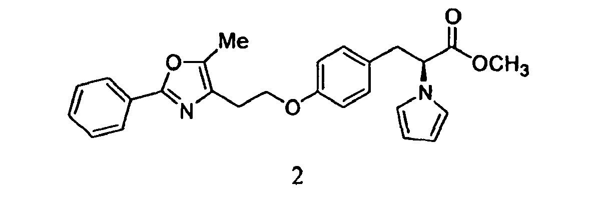

**General Coupling Procedure (General Procedure B)**. Pyrrolotyrosine methyl ester (1) (2.68 g, 10.9 mmol), 2-(5-Methyl-2-phenyloxazol-4-yl) ethanol (2.22g, 10.9 mmol) and triphenylphosphine (3.15g, 12.0 mmol) were dissolved in anhydrous THF (100 mL) under N₂ at 0 °C. A solution of diethyl azodicarboxylate (DEAD) (1.89 mL, 12.0 mmol) in THF (50 mL) was added slowly over 30 min. and allowed to equilibrate to 23 °C over 18 hr. The solution was diluted with EtOAc (50mL) and washed with water (30 mL), brine (30 mL) and dried over MgSO₄. After removal of solvent *in vacuo,* SiO₂ gel chromatography with 10%EtOAc/Hex gave 2.4 g of 2 in 51 % as a clear oil. Low Resolution Mass Spectroscopy (LRMS) C₂₆H₂₆N₂O₄ MW=430.501 calc. M+1 = 431.2 obs. ¹H NMR (400MHz) CDCl₃ δ: 7.95 (2H, d, *J*=5.8Hz), 7.40 (3H, m), 6.85 (2H, d, *J*=8.3 Hz), 6.72 (2H, d, *J*=8.5 Hz), 6.66 (2H, s), 6.10 (2H, s), 4.63 (1H, dd, *J*=6.83, 8.30 Hz), 4.16 (2H, t, 6.6, 6.6 Hz), 3.65 (3H, s), 3.29 (1H, dd, *J*=6.3, 6.3 Hz), 3.13 (1H, dd, *J*=8.8, 8.8 Hz), 2.93 (2H, t, *J*=6.6, 6.6 Hz), 2.32 (3H, s).

### (b) Pyrrolotyrosine methyl ester (1).

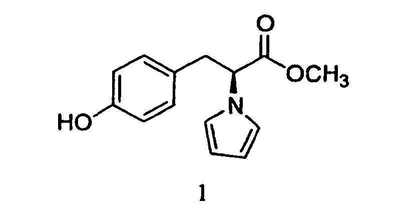

S-tyrosine methyl ester (25.6 g, 131 mmol), 2,5-dimethoxy-tetrahydrofuran (17 mL, 223 mmol) and NaOAc (21.5 g, 262 mmol) were dissolved in a 1:1 mixture of H₂O and acetic acid (150 mL:150 mL) and stirred until homogeneous (ca. 30 min.). The temperature was then raised to 100°C for 20 min. in an oil bath during which time the solution turned a dark brown color. After the indicated period of time, the solution was removed from the hot bath and allowed to cool to 23°C. The reaction mixture was diluted with H₂O (100 mL) and and extracted with EtOAc (3 x 75 mL). The organic layers were combined and washed consecutively with H₂O (2 x 75 mL), brine (50 mL) and dried over Mg₂SO₄. The solvent was removed *in vacuo* and the crude residue was chromatographed with 5% MeOH/CHCl₃ to give 15 g (35% yield) of cream colored crystals. ¹H NMR (400MHz) δ (CDCl₃) 6.83 (2H, d, 8 Hz), 6.67 (2H, d, 2Hz), 6.65 (2H, d, 8Hz), 6.11 (2H, d, 2Hz), 4.65 (1H, q, 6Hz), 4.66 (1H, s), 3.66 (3H, s), 3.31 (1H, dd, 6 Hz), 3.28 (1H, dd, 6 Hz).

The procedures described in Example 1 were used to prepare (R)-3-{4-[2-(5-Methyl-2-phenyl-oxazol-4-yl)-ethoxy]-phenyl}-2-pyrrol-1-yl-propionic acid and racemic (S)-3-{4-[2-(5-Methyl-2-phenyl-oxazol-4-yl)-ethoxy]-phenyl}-2-pyrrol-1-yl-propionic acid.

### Example 2

### (d) Benzoic acid 4-(2-methoxycarbonyl-2-pyrrol-1-yl-ethyl)-phenyl ester (4).

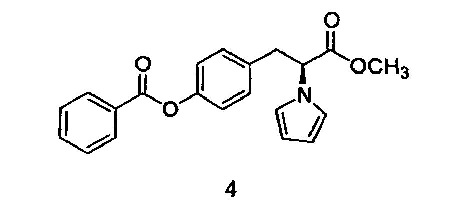

**General Procedure E.** Triethylamine (290 µL, 2.06 mmol) was added to a stirring solution of Pyrrolotyrosine methyl ester (252 mg, 1.03 mmol) in dichloromethane (20 mL) under N₂ at 23°C. Benzoyl chloride (143 µL, 1.23 mmol) was added dropwise over a 5 minute period and the reaction was stirred for 2 hours. Solvent was removed *in vacuo* and chromatographed via SiO₂ with 10% EtOAc/hexanes to give the benzoyl ester 4 in 86% yield (310 mg, 0.89 mmol) as a white foam. C₂₁H₁₉NO₄ Mass Calc. = 349.380; M+1 (obs)= 350.2. ¹H NMR (400MHz, CDCl₃) δ: 8.17 (2H, d, *J*=6.35 Hz), 7.62 (1H, m), 7.47 (2H, m), 7.09 (2H, d, *J*=8.8Hz), 7.04 (2H, d, *J*=8.6Hz), 6.71 (2H, t, *J*=2.2, 2.1 Hz), 6.16 (2H, t, *J*=2.2, 2.2 Hz), 4.72 (1H, dd, *J*=2.4, 2.4 Hz), 3.70 (3H, s), 3.43 (1H, dd, *J*=6.3, 6.3 Hz), 3.25 (1H, dd, *J*=9.0, 9.0 Hz).

### Example 3

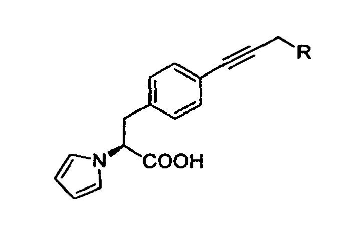

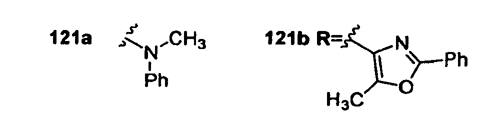

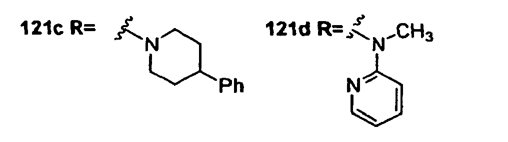

### General procedure for the preparation of analogs 121 a-d.

(*S*)-3-{4-[3-(Methyl-phenyl-amino)-prop-1-ynyl]-phenyl}-2-pyrrol-1-yl-propionic acid (121a). Ester 120a (0.82 g, 2.201 mmol) was dissolved in THF:H₂O (40:10 mL) and LiOH·H₂O (0.138 g, 3.301 mmol) was added and the mixture was stirred at room temperature for 3.5 hours. The solvent was removed and the residue was diluted with water, acidified with 10% HCl. The mixture was extracted with CHCl₃; (3 × 50 mL). The combined organic extracts were dried over MgSO₄, filtered and the solvent removed. Purification by chromatography on silica gel eluting with 0-2% MeOH in CHCl₃ containing formic acid (0-0.1 %) gave pure 129a as a light brown solid (0.727 g, 92%): Mp 55°C to 60°C; ¹H NMR (CDCl₃, 400 MHz) δ 7.29-7.25 (m, 2 H), 7.21 (d, *J*= 8.0 Hz, 2 H), 6.92 (d, *J*= 8.0 Hz, 2 H), 6.88 (d, *J =* 8.4 Hz, 2 H), 6.83 (t, *J =* 7.4 Hz, 1H), 6.65 (t, *J*= 2.0 Hz, 2 H), 6.13 (t, *J* = 2.0 Hz, 2 H), 4.72 (dd, *J* = 9.2 and 6.0 Hz, 1 H), 4.23 (s, 2 H), 3.41-3.20 (m, 2 H), 3.00 (s, 3 H); CIMS *m*/*z* 359 (M + H)⁺; IR 3418, 2960, 1726, 1597, 1272 cm⁻¹. Anal. calcd for C₂₃H₂₂N₂O₂·0.3 H₂O: C, 75.93; H, 6.26; N, 7.74. Found: C, 75.73; H, 6.19; N, 7.53.

(*S*)-3-{4-[3-(5-Methyl-2-phenyl-oxazol-4-yl)-prop-1-ynyl]-phenyl}-2-pyrrol-1-yl-propionic acid (121b). Prepared from methyl ester 120b (0.337 g, 0.794 mmol) by the general procedure described for 129a. Purification by chromatography on silica gel eluting with hexanes:ethyl acetate (2:1) followed by hexanes:ethyl acetate (2:1) containing 0.2% formic acid gave acid 128b as a pale yellow solid (0.231 g, 71 %): mp 178°C to 180°C; ¹H NMR (CDCl₃, 400 MHz) δ 7.78-7.76 (m, 2 H), 7.22 (m, 3 H), 7.05 (d, *J* = 8.0 Hz,2 H), 6.72 (d, *J* = 8.4 Hz, 2 H), 6.46 (t, *J*= 2.0 Hz, 2 H), 5.86 (t, *J*= 2.0 Hz, 2 H), 4.48 (dd, *J*= 9.6 and 5.6 Hz, 1 H), 3.50 (s, 2 H), 3.27-2.97 (m, 2 H), 2.26 (s, 3 H); CIMS *m*/*z* 409 (M - H)⁺. Anal. calcd for C₂₆H₂₂N₂O₃: C, 76.08; H, 5.40; N, 6.82. Found: C, 75.77; H, 5.45; N, 6.70.

(*S*)-3-{4-[3-(4-Phenyl-piperidin-1-yl)-prop-1-ynyl]-phenyl}-2-pyrrol-1-yl-propionic acid (121c). Prepared from ester 120c (0.344 g, 0.806 mmol) following the general procedure described for 129a. Purification by chromatography eluting with 0-8% MeOH in CHCl₃ containing 0.1% formic acid gave pure 129c as a white solid (0.307 g, 92%): Mp 201 °C-203°C; ¹H NMR (CDCl₃, 400 MHz) δ 7.33-21 (m, 5 H), 7.17 (d, *J*= 7.3 Hz, 2 H), 7.01 (d, *J*= 8.3 Hz, 2 H), 6.70 (t, *J*= 2.2 Hz, 2 H), 6.08 (t, *J=* 2.2 Hz, 2 H), 4.68 (dd, *J=* 8.8 and 6.1 Hz, 1H), 3.91 (s, 2 H), 3.48-3.20 (m, 4 H), 2.98 (t, J= 11.5 Hz, 2 H), 2.61 (t, *J*= 11.9 Hz, 1 H), 2.25-2.13 (m, 2 H), 1.96 (d, *J* = 12.7 Hz, 2 H); CIMS *m*/*z* 413 (M + H)⁺. Anal. calcd for C₂₇H₂₈N₂O₂·0.9 H₂O: C, 75.64; H, 7.01; N, 6.53. Found: C, 75.29; H, 6.75; N, 6.44.

(*S*)-3-{4-[3-(Methyl-pyridin-2-yl-amino)-prop-1-ynyl]-phenyl}-2-pyrrol-1-yl-propionic acid (121d). Prepared from ester 120d (0.110 g, 0.294 mmol) by the general procedure described above. Acid 129d was obtained as a yellow solid (0.098 g, 93%): ¹H NMR (CDCl₃, 400 MHz) δ 8.02 (m, 1 H), 7.51-7.46 (m, 1 H), 7.14 (d, *J*= 8.3 Hz, 2 H), 6.82 (d, *J*= 8.0 Hz, 2 H), 6.62-6.54 (m, 2 H), 6.58 (t, *J*= 2.0 Hz, 2 H), 6.00 (t, *J* = 2.2 Hz, 2 H), 4.61 (dd, *J* = 9.4 and 5.6 Hz, 1H), 4.37 (s, 2 H), 3.35-3.10 (m, 2 H), 3.05 (s, 3 H); CIMS *m*/*z* 360 (M + H)⁺. Anal. calcd for C₂₂H₂₁N₃O₂·0.5C₄H₈O₂: C, 71.44; H, 6.25; N, 10.41. Found: C, 71.14; H, 6.07; N, 10.20.

The ester starting materials were prepared in the following manner.

### (a) (120 a-d).

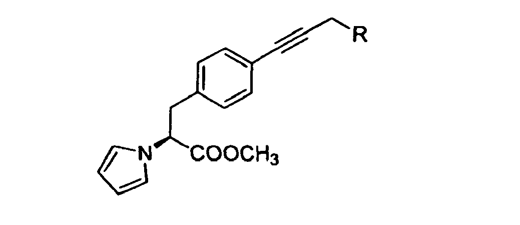

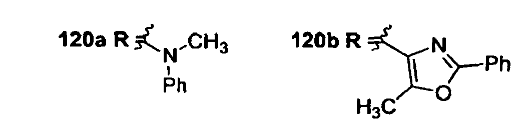

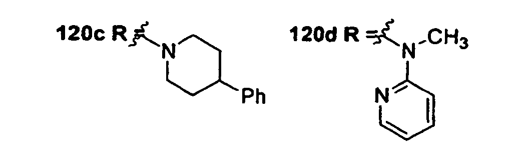

### General procedure for the synthesis of esters 120 a-d.

(*S*)-3-{4-[3-(Methyl-phenyl-amino)-prop-1-ynyl]-phenyl}-2-pyrrol-1-yl-propionic acid methyl ester (120a). Triflate 119 (1.66 g, 4.399 mmol) and Pd(PPh₃)₄ (0.356 g, 0.308 mmol) were dissolved in dry DMF (10 mL). A solution of *N*-methyl-*N*-prop-2-ynyl aniline (Magnus, P.; Ladlow, M.; Elliot, *J*.; Sook Kim, C. *J*. *Chem. Soc. Chem. Comm.* **1989**,518-519) (1.27 g, 8.798 mmol) in DMF (2 mL) was added, followed by triethylamine (1.84 mL, 13.197 mmol). Nitrogen was passed through the reaction mixture for 0.5 hr. CuI (0.167 g, 0.880 mmol) was added and the mixture heated at 45-50 °C for 6 h under nitrogen. At this time additional Pd(PPh₃)₄ (0.356 g, 0.308 mmol) was incorporated. Heating was continued for 14 h and additional *N*-methyl-*N*-prop-2-ynyl aniline (0.635 g, 4.399 mmol) and catalyst (0.254 g, 0.220 mmol) were added. The mixture was heated for another 12 hr. At this time the mixture was allowed to cooled and diluted with water (150 mL) and Et₂O (100 mL). The phases were separated and the aqueous phase was extracted with Et₂O (4 × 80 mL). The combined organic extracts were washed with water and brine. Activated carbon was added and the mixture was boiled for 15-20 min. It was dried under MgSO₄, filtered and the solvent removed. Purification by flash chromatography on silica gel eluting with 60%-0% petroleum ether in dichloromethane followed by a second chromatographic purification eluting with hexanes:ethyl acetate (7:1 to 5:1) gave pure 11 as a thick oil (1.256 g, 77%): ¹H NMR (CDCl₃, 400 MHz) δ 7.29-7.21 (m, 4 H), 7.23 (d, *J* = 8.0 Hz, 2 H), 6.92-6.66 (m, 1H), 6.89 (d, *J*= 8.4 Hz, 2 H), 6.66 (t, *J =* 2.4 Hz, 2 H), 6.12 (t, *J =* 2.4 Hz, 2 H), 6.68 (dd, *J =* 9.2 and 6.4 Hz, 1 H), 4.24 (s, 2 H), 3.67 (s, 3 H), 3.39-3.18 (m, 2 H), 3.02 (s, 3 H); CIMS *mlz* 373 (M + H)⁺.

(S)-3-{4-[3-(5-Methyl-2-phenyl-oxazol-4-yl)-prop-1-ynyl]-phenyl}-2-pyrrol-1-yl-propionic acid methyl ester (120b). This compound was prepared from triflate 119 (1.47 g, 3.9 mmol) and 5-methyl-2-phenyl-4-prop-2-ynyloxazole 125 (1.0 g, 5.070 mmol) following the general procedure described for 128a, except that no additional catalyst or 127 was added and the reaction was carried out at 90°C. Purification by chromatography eluting with hexanes:ethyl acetate (5:1 to 4:1) gave pure 128b as a thick oil (1.20, 73%): ¹H NMR (CDCl₃, 400 MHz) δ 7.99 (d, *J*= 8.4 Hz, 1 H), 7.98 (d, *J*= 7.2 Hz, 1 H), 7.45-7.39 (m, 3 H), 7.29 (d, *J*= 8.0 Hz, 2 H), 6.92 (d, *J* = 8.4 Hz, 2 H), 6.68 (t, *J*= 2.0 Hz, 2 H), 6.13 (t, *J* = 2.0 Hz, 2 H), 4.70 (dd, *J* = 8.8 and 6.4 Hz, 1 H), 3.71 (s, 2 H), 3.69 (s, 3 H), 3.40-3.20 (m, 2 H), 2.46 (s, 3 H); CIMS *m*/*z* 425 (M + H)⁺.

(*S*)-3-{4-[3-(4-Phenyl-piperidin-1-yl)-prop-1-ynyl]-phenyl}-2-pyrrol-1-yl-propionic acid methyl ester (120c). This compound was prepared from triflate 119 (0.500 g, 1.325 mmol) and *N*-prop-2-ynyl-4-phenylpiperidine (Lambert, S. *J*.; Kabalka, G. W.; Knapp, F. F. Jr.; Srivastava, P. C. *J. Org. Chem.* **1991*****,** 56,* 3707-3711) (0.396 g, 1.987 mmol) following the general procedure described for 128a with the exception that the reaction was carried out at 85°C. Additional catalyst (5 mol%) and *N*-prop-2-ynyl-4-phenylpiperidine (0.132 g, 0.662 mmol) were added after 20 hours. Purification by flash chromatography on silica gel eluting with 35%-45% ethyl acetate in hexanes afforded pure 128c as a thick oil (0.344 g, 61%): ¹H NMR (CDCl₃, 400 MHz) δ 7.32-7.17 (m, 5 H), 7.31 (d, *J* = 8.4 Hz, 2 H), 6.93 (d, *J* = 8.0 Hz, 2 H), 6.69 (t, *J* = 2.0 Hz, 2 H), 6.14 (t, *J* = 2.0 Hz, 2 H), 4.71 (dd, *J* = 9.2 and 6.4 Hz, 1 H), 3.70 (s, 3 H), 3.53 (s, 2 H), 3.41- 3.21 (m, 2 H), 3.08 (bd, *J* = 11.6 Hz, 2 H), 2.55-2.46 (m, 1 H), 2.36 (t, *J* = 11.2 Hz, 1 H), 2.35 (t, *J* = 11.2 Hz, 1 H), 1.91-1.79 (m, 4 H); CIMS *m*/*z* 427 (M + H)⁺.

(*S*)-3-{4-[3-(Methyl-pyridin-2-yl-amino)-prop-1-ynyl]-phenyl}-2-pyrrol-1-yl-propionic acid methyl ester (120d). This compound was prepared from triflate 119 (0.526 g, 1.394 mmol) and 2-(*N*-methyl-*N*-prop-2-ynyl)pyridine (see 126, below) (0.407 g, 2.788 mmol) following the general procedure described for 128a, with the exception that piperidine was used as solvent instead of DMF. Purification by chromatography on silica gel eluting with hexanes:ethyl acetate (4:1) gave 128d as a yellowish thick oil (0.132 g, 25.3%): ¹H NMR (CDCl₃, 400 MHz) δ 8.19-8.17 (m, 1 H), 7.48-7.43 (m, 1H), 7.22 (d, *J*= 8.0 Hz, 2 H), 6.87 (d, *J =* 8.0 Hz, 2 H), 6.64 (t, *J*= 2.0 Hz, 2 H), 6.59 (d, *J* = 7.6 Hz, 1 H), 6.58 (d, *J =* 6.4 Hz, 1H), 6.09 (t, *J=* 2.0 Hz, 2 H), 4.66 (dd, *J=* 9.0 and 6.4 Hz, 1H), 4.54 (s, 2 H), 3.65 (s, 3 H), 3.36-3.16 (m, 2 H), 3.09 (s, 3 H); CIMS *m*/*z* 374 (M + H)⁺.

(b) Triflate 119, used in the coupling action above, was prepared in the following manner.

### (S)-2-Pyrrol-1-yl-3-[(4-trifluoromethanesulfonyloxy)phenyl]-propionic acid methyl ester (119).

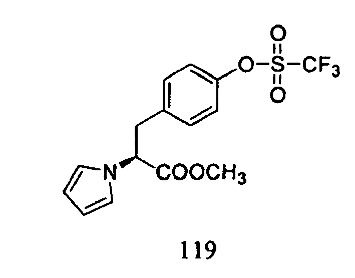

A mixture of phenol 1(See Example 1) (6.64 g, 27.088 mmol) and N-phenyltrifluoromethanesulfonimide (10.47 g, 27.9 mmol) in CH₂Cl₂ (70 mL) under nitrogen was cooled at 0°C. Triethylamine (4.15 mL, 29.8 mmol) was added slowly. The mixture was stirred at 0°C for 1 hr. Then the temperature was allowed to reach room temperature slowly and stirred at this temperature for 2.5 hr. The mixture was diluted with Et₂O (70 mL) and washed with water, 1 N NaOH, and brine. The organic phase was dried over MgSO₄, filtered, and the solvent removed. Purification by flash chromatography on silica gel eluting with hexanes:ethyl acetate (5:1) afforded 127 as a thick oil (9.55 g, 93%) which solidified upon cooling and trituration to give a white solid: [α]²⁵_{D} -93.6° (*c* = 5, CHCl₃); ¹H NMR (CDCl₃, 400 MHz) δ 7.13 (d, *J*= 8.8 Hz, 2 H), 7.02 (d, J= 8.8 Hz, 2 H), 6.66 (t, *J* = 2.1 Hz, 2 H), 6.14 (t, *J* =2.1 Hz, 2 H), 4.69 (dd, *J =* 9.3 and 5.9 Hz, 1 H), 3.72 (s, 3 H), 3.43-3.25 (m, 2 H); IR 1731, 1426, 1203, 1136 cm⁻¹; CIMS *m*/*z* 378 (M + H)⁺. Anal. calcd for C₁₅H₁₄F₃NO₅S: C, 47.75; H, 3.74; N, 3.71. Found: C, 47.83; H, 3.64; N, 3.54.

(c) The alkynes that were used in the coupling reaction above were prepared in the following manner.

### 2-(N-Methyl-N-prop-2-ynyl)pyridine (118).

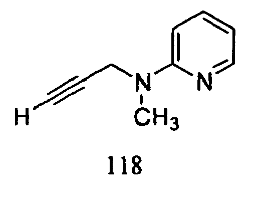

Sodium hydride (0.467 g, 11.673 mmol) was suspended in DMF (10 mL) under nitrogen and stirred in an ice bath. 2-(Methylamino)pyridine (1 mL, 9.728 mmol) was added and the mixture stirred at 0°C for 45 min. An 80% solution of propargyl bromide in toluene (1.19 mL, 10.7 mmol) was then incorporated. The mixture was allowed to reach room temperature and stirred overnight. The mixture was diluted with water and extracted with Et₂O. The combined organic extracts were washed with water and brine, dried over MgSO₄, filtered, and the solvent removed. Purification by flash chromatography on silica gel eluting with hexanes:ethyl acetate (8:1) afforded 126 as an oil (0.867 g, 61 %): ¹H NMR (CDCl₃, 400 MHz) δ 8.17 (dd, *J=* 4.6 and 1.7 Hz, 1H), 7.46 (dt, *J=* 8.5 and 2.0 Hz, 1 H), 6.58 (m, 2 H), 4.35 (d, *J* = 2.4 Hz, 2 H), 3.04 (s, 3 H), 2.11 (t, *J* = 2.4 Hz, 1 H); CIMS *m*/*z* 147 (M + H)⁺.

### 5-Methyl-2-phenyl-4-prop-2-ynyloxazole (117).

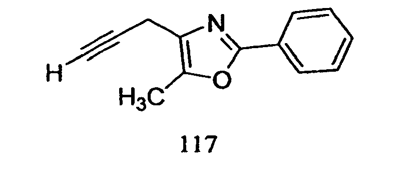

According to the procedure of Hulin (Hulin, B.; Newton, L. S.; Lewis, D. M.; Genereux, P. E.; Gibbs, M.; Clark, D. A. *J. Med. Chem.* **1996**, *39,* 3897-3907). Alkyne 7 (3.01 g, 10.472 mmol) was dissolved in MeOH (150 mL) and treated with 10% KOH (10 mL). The mixture was stirred at room temperature for 4.5 hours. At this time the solvents were removed and the residue diluted with water and acidified to pH~2 with 6 M HCl. The solid that precipitated was separated by vacuum filtration and dried. The filtrate was extracted with ethyl acetate (3 × 40 mL) and the combined organic extracts were dried over MgSO₄, filtered, and the solvent removed. The solid obtained from the previous steps (2.19 g) was treated with TFA (16 mL) and TFAA (8 mL) at 35-40 °C overnight, as previously described by Hulin and collaborators, to give oxazole 125 after purification by flash chromatography over silica gel eluting with hexanes:ethyl acetate (10:1 to 9:1). Oxazole 125 was obtained as an off-white solid (1.89 g, 94%): ¹H NMR (CDCl₃, 400 MHz) δ 7.96-7.99 (m, 2 H), 7.37-7.44 (m, 3 H), 3.50 (d, *J=* 2.8 Hz, 2 H), 2.41 (s, 3 H), 2.12 (t, *J* = 2.8 Hz, 1 H); CIMS *m*/*z* 198 (M + H)⁺.

### N-[(1-acetyl-4-(trimethylsilyl)but-3-ynyl]benzamide (116).

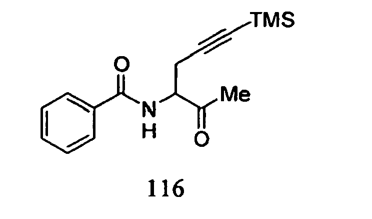

Amide 123 (2.55 g, 14.4 mmol) was dissolved in THF (150 mL) and cooled to -78 °C under nitrogen. A 1.0 M solution of LHMDS in THF (14.4 mL, 14.4 mmol) was added and the mixture stirred for 0.5 hr. A solution of 3-bromo-1-(trimethylsilyl)-1-propyne (2.6 mL, 18.7 mmol) in THF (15 mL) was added. The mixture was allowed to warm to room temperature and stirred overnight. The mixture was diluted with water and the phases were separated. The aqueous phase was extracted with ethyl acetate (3 × 50 mL) and the combined organic extracts were dried over MgSO₄, filtered, and the solvent removed. Purification by flash chromatography on silica gel eluting with hexanes:ethyl acetate (3:1) gave amide 124 as white solid (3.01 g, 73%): ¹H NMR (CDCl₃, 400 MHz) δ 7.68 (d, *J* = 7.2 Hz, 2 H), 7.40 (t, *J =* 7.2 Hz, 1 H), 7.32 (t, *J =* 7.2 Hz, 2 H), 7.02 (bd, *J =* 6.4 Hz, 1 H), 4.71 (q, *J =* 5.2 Hz, 1 H), 2.78 (d, *J =* 5.6 Hz, 2 H), 2.20 (s, 3 H), 0.01 (s, 9 H); CIMS *m*/*z* 288 (M + H)⁺; IR 3396, 2961, 2174, 1722, 1644, 1481, 1250 cm⁻¹. Anal. calcd for C₁₆H₂₁NO₂Si: C, 66.86; H, 7.36; N, 4.87. Found: C, 67.15; H, 7.49; N, 4.72.

### Benzamidoacetone (115).

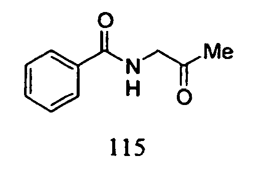

According to the procedure of Ellinger (Ellinger, L. P.; Goldberg, A. A. *J Chem. Soc.* **1949**, 263). *N*-(2-Hydroxypropyl)benzamide (123) (3.0 g, 16.7 mmol) was dissolved in CH₂Cl₂ (60 mL) and PDC (9.42 g, 25.0 mmol) was added. The mixture was stirred at room temperature for 24 and more PDC (9.42 g, 25.0 mmol) was added. Stirring was continued for 24 hours. The mixture was diluted with ethyl acetate and filtered through a celite pad. The residue was then passed through a shoroom temperature silica gel column eluting with ethyl acetate. The solvent was removed and the residue was purified by flash chromatography on silica gel eluting with hexanes:ethyl acetate (1:1) containing MeOH (0 to 4%). This purification afforded amide 122 as a white solid (2.21 g, 75%): ¹H NMR (CDCl₃, 400 MHz) δ 7.76 (dd, *J*= 7.0 and 1.5 Hz, 2 H), 7.48-7.36 (m, 3 H), 6.96 (bs, 1H), 4.30 (d, *J=* 4.6 Hz, 2 H), 2.21 (s, 3 H); CIMS *mlz* 178 (M + H)⁺.

### N-(2-Hydroxypropyl)benzamide (114).

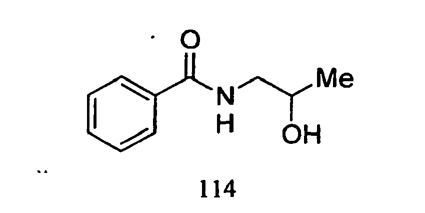

According to the procedure of Arai (Arai, K.; Tamura, S.; Masumizu, T.; Kawai, K.-I.; Naka*J*ima, S.; Ueda, A. *Can. J. Chem.* **1990,** *68,* 903-907). A solution of DL-1-amino-2-propanol (3.4 mL, 43.2 mmol) and triethylamine (16.4 mL, 117.9 mmol) in CH₂Cl₂ (60 mL) under nitrogen was cooled at -78°C. Benzoyl chloride (4.6 mL, 39.3 mmol) was added dropwise. The mixture was allowed to warm slowly and stirred at room temperature overnight. It was then diluted with CH₂Cl₂ (100 mL) and washed with cold 5% HCl and brine. The phases were separated and the aqueous phase was extracted with CH₂Cl₂ (2 × 50 mL). The combined organic extracts were dried over MgSO₄, filtered, and the solvent removed. The residue was dried under vacuum to give amide 5 as a pale yellow solid (6.21 g, 88%): ¹H NMR (CDCl₃, 400 MHz) δ 7.75 (d, *J=* 6.8 Hz, 2 H), 7.49-7.39 (m, 3 H), 6.57 (bs, 1 H), 4.00 (m, 1H), 3.67-3.61 (m, 1 H), 3.31-3.24 (m, 1H), 1.22 (d, *J* = 6.3 Hz, 3 H).

### Example 4

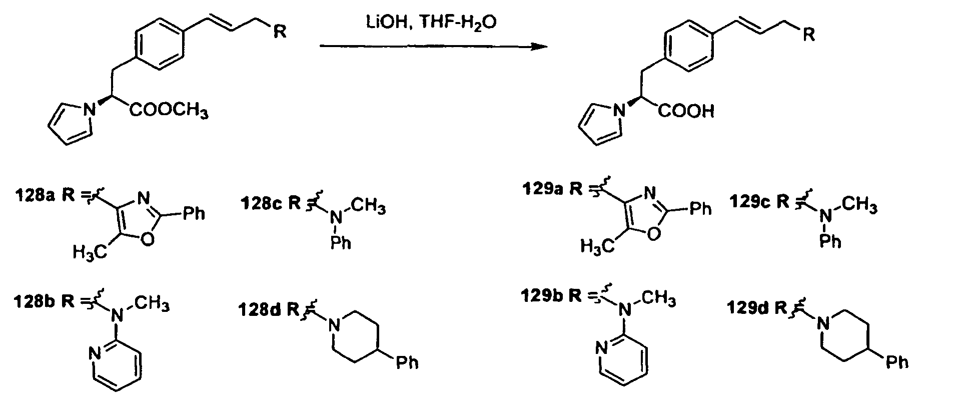

### General procedure for the preparation of analogs 129a-d.

(*S*)-3-{4-[(*E*)-3-(5-Methyl-2-phenyl-oxazol-4-yl)-propenyl]-phenyl}-2-pyrrol-1-yl-propionic acid (129a). Prepared from ester 128a (0.140 g, 0.328 mmol) following the general procedure. Purification by chromatography on silica gel eluting with hexanes:ethyl acetate (2:1) containing formic acid (0-0.2%) gave acid 138a as an off-white solid (0.085 g, 63%): Mp 132°C-133°C; ¹H NMR (CDCl₃, 400 MHz) δ 7.95-7.92 (m, 2 H), 7.41-7.39 (m, 3 H), 7.19 (d, J= 8.0 Hz, 2 H), 6.90 (d, *J =* 8.4 Hz, 2 H), 6.65 (t, *J* = 2.0 Hz, 2 H), 6.37 (d, *J* = 15.6 Hz, 1 H), 6.25 (dt, *J =* 16.0 and 6.4 Hz, 1 H), 6.13 (t, *J =* 2.0 Hz, 2 H), 4.57 (dd, *J =* 8.8 and 6.0 Hz, 1 H), 3.39 (d, J= 6.8 Hz, 2 H), 3.38-3.15 (m, 2 H), 2.33 (s, 3 H); CIMS *m*/*z* 413 (M + H)⁺. Anal. calcd for C₂₆H₂₄N₂O₃·0.1 H₂O: C, 75.38; H, 5.89; N, 6.76. Found: C, 75.58; H, 6.21; N, 6.37.

(S)-3-{4-[(*E*)-3-(Methyl-pyridin-2-yl-amino)-propenyl]-phenyl}-2-pyrrol-1-yl-propionic acid (129b). Prepared from ester 128b (0.481 g, 1.281 mmol) following the general procedure described above. Purification by chromatography on silica gel gel eluting with 0-15% MeOH in CHCl₃ followed by THF gave pure 138b (0.123 g, 26%): Mp 155°C-165°C; ¹H NMR (CD₃OD, 400 MHz) δ 7.98 (dd, *J =* 5.2 and 1.0 Hz, 1 H), 7.52-7.47 (m, 1 H), 7.13 (d, *J =* 8.4 Hz, 2 H), 6.91 (d, *J =* 8.4 Hz, 2 H), 6.66 (t, *J* = 2.0 Hz, 2 H), 6.56 (dd, *J* = 6.4 and 5.2 Hz, 1 H), 6.38 (d, *J =* 15.6 Hz, 1H), 6.14 (dt, *J =* 16.0 and 5.6 Hz, 1 H), 5.92 (t, *J* = 2.0 Hz, 2 H), 4.58 (dd, *J* = 9.6 and 5.6 Hz, 1 H), 4.22 (dd, *J =* 5.6 and 1.2 Hz, 2 H), 3.34-3.08 (m, 2 H), 3.02 (s, 3 H); CIMS *m*/*z* 362 (M + H)⁺.

(*S*)-3-{4-[(*E*)-3-(Methyl-phenyl-amino)-propenyl]-phenyl}-2-pyrrol-1-yl-propionic acid (129c). Ester 128c (0.877 g, 2.342 mmol) was dissolved in THF:H₂O (40:10 mL) and LiOH·H₂O (0.147 g, 3.513 mmol) was added and the mixture was stirred at room temperature for 3.5 hours. The solvent was removed and the residue was diluted with water, acidified with 10% HCl. The mixture was extracted with CHCl₃ (3 × 50 mL). The combined organic extracts were dried over MgSO₄, filtered and the solvent removed. Purification by chromatography on silica gel eluting with 0-3% MeOH in CHCl₃ containing 0.1% formic acid gave pure 138c as brownish solid (0.280 g, 33%): mp 85°C-90°C; ¹H NMR (CDCl₃, 400 MHz) δ ***E*-isomer:** 7.26-7.18 (m, 3 H), 6.92 (d, *J=* 8.4 Hz, 2 H), 6.81-6.69 (m, 4 H), 6.68 (t, *J=* 2 Hz, 2 H), 2 H), 6.44 (d, *J=* 15.6 Hz, 1 H), 6.14 (t, *J*= 2.0 Hz, 2 H), 4.73 (dd, *J =* 9.2 and 6.0 Hz, 1 H), 4.03 (dd*, J* = 5.6 and 1.2 Hz, 2 H), 3.48-3.24 (m, 2 H), 2.97 (s, 3 H); CIMS *mlz* 361 (M + H)⁺. Anal. calcd for C₂₃H₂₄N₂O₂·0.9 H₂O: C, 74.41; H, 6.84; N, 7.55. Found: C, 74.03; H, 6.62; N, 7.36.

(*S*)-3-{4-[(*E*)-3-(4-Phenyl-piperidin-1-yl)-propenyl]-phenyl}-2-pyrrol-1-yl-propionic acid (129d). Prepared from ester 128d (1.772 g, 4.135 mmol) following the general procedure. Purification by chromatography on silica gel eluting with 0-12% MeOH in CHCl₃ containing formic acid (0%-0.1 %) gave acid 138d as a pale yellow foam (1.38 g, 80%): Mp 126°C-130°C; ¹H NMR (CDCl₃, 400 MHz) δ 7.30-7.18 (m, 5 H), 7.17 (d, *J* = 7.1 Hz, 2 H), 6.95 (d, *J*= 8.1 Hz, 2 H), 6.57 (d, J= 16.0 Hz, 1 H), 6.55 (t, *J=* 2.0 Hz, 2 H), 6.26 (dt, *J=* 15.6 and 7.3 Hz, 1 H), 6.02 (t, *J=* 2.0 Hz, 2 H), 4.56 (dd, *J=* 8.8 and 6.1 Hz, 1H), 3.69-3.49 (m, 4H), 3.42-3.12 (m, 2 H), 2.70-2.62 (m, 3 H), 2.33-2.26 (m, 2 H), 1.91 (m, 2 H); CIMS *m*/*z* 415 (M + H)⁺. Anal. calcd for C₂₇H₃₀N₂O₂·1.0CH₂O₂: C, 73.02; H, 7.00; N, 6.08. Found: C, 72.85; H, 7.05; N, 6.06.

The ester starting materials were prepared in the following manner. Esters 128a-b.

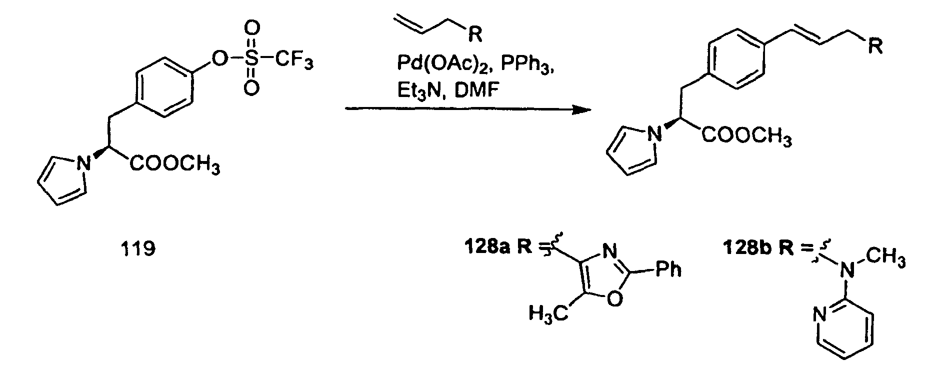

(*S*)-3-{4-[(*E*)-3-(5-Methyl-2-phenyl-oxazol-4-yl)-propenyl]-phenyl}-2-pyrrol-1-yl-propionic acid methyl ester (128a). A mixture of triflate 119 (Example 3) (1.5 g, 3.975 mmol), 5-methyl-2-phenyl-4-prop-2-enyloxazole 135 (1.19 g, 5.962 mmol), Pd(OAc)₂ (0.045 g, 0.199 mmol), PPh₃ (0.114 g, 0.437 mmol), triethylamine (1.10 mL, 7.95 mmol) was dissolved in DMF (15 mL). Nitrogen was passed through the mixture for 20-25 min. The reaction mixture was heated at 90°C under nitrogen for 44 hours. The mixture was allowed to cool and filtered through a celite pad, washing with ethyl ether. Water was added and the phases were separated. The aqueous phase was extracted with ethyl ether (4 × 60 mL). The combined organic extracts were washed with water, brine, dried over MgSO₄, filtered and the solvent removed. Purification by chromatography on silica gel eluting with 20%-25% ethyl acetate in hexanes afforded 137a as a thick oil (0.668, 39%): ¹H NMR (CDCl₃, 400 MHz) δ 8.00-7.97 (m, 2 H), 7.44-7.39 (m, 3 H), 7.22 (d, *J =* 8.0 Hz, 2 H), 6.91 (d, *J =* 8.0 Hz, 2 H), 6.69 (t, *J* = 2.0 Hz, 2 H), 6.42 (d, *J =* 14.8 Hz, 1 H), 6.32 (dt, *J =* 15.6 and 6.4 Hz, 1 H), 6.13 (t, *J =* 2.0 Hz, 2 H), 4.71 (dd, *J=* 8.8 and 6.4 Hz, 1 H), 3.68 (s, 3 H), 3.42 (d, *J =* 6.4 Hz, 2 H), 3.39-3.18 (m, 2 H), 2.34 (s, 3 H); CIMS *mlz* 427 (M + H)⁺.

(*S*)-3-{4-[(*E*)-3-(Methyl-pyridin-2-yl-amino)-propenyl]-phenyl}-2-pyrrol-1-yl-propionic acid methyl ester (128b). This compound was prepared from triflate 119 (1.0 g, 5.30 mmol) and 2-(*N*-methyl-*N*-prop-2-enyl)pyridine 136 (0.785 g, 5.300 mmol) following the procedure described for 137a. Additional Pd(OAc)₂ (5 mol%) was added after 16 hr. The reaction was completed after 24 hours. Purification by chromatography eluting with 20%-25% ethyl acetate in hexanes gave 137b as a thick oil (0.600, 60%): ¹H NMR (CDCl₃, 400 MHz) δ 8.18-8.16 (m, 1H), 7.47-7.42 (m, 1H), 7.21 (d, *J*= 8.4 Hz, 2 H), 6.92 (d, *J*= 8.0 Hz, 2 H), 6.69 (t, *J* = 2.0 Hz, 2 H), 6.67-6.51 (m, 2 H), 6.42 (d, *J* = 15.6 Hz, 1 H), 6.18 (dt, *J=* 16.0 and 5.6 Hz, 1 H), 6.13 (t, *J*= 2.0 Hz, 2 H), 4.71 (dd, *J=* 8.6 and 6.0 Hz, 1 H), 4.30 (dd, *J* = 5.6 and 1.2 Hz, 2 H), 3.66 (s, 3 H), 3.39-3.19 (m, 2 H), 3.05 (s, 3 H); CIMS *m*/*z* 376 (M + H)⁺.

### 2-(N-Methyl-N-prop-2-enyl)pyridine (127).

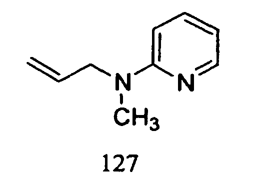

Sodium hydride (0.82 g, 20.4 mmol) was suspended in DMF (10 mL) under nitrogen and stirred in an ice bathr. 2-(Methylamino)pyridine (1.5 mL, 14.6 mmol) was added. The ice bath was removed and the mixture stirred at room temperature for 0.5 hr. The mixture was cooled back in an ice bath and allyl bromide (1.9 mL, 21.9 mmol) was added. The mixture was allowed to reach room temperature and stirred overnight. The mixture was diluted with water and extracted with Et₂O. The combined organic extracts were washed with water and brine, dried over MgSO₄, filtered, and the solvent removed. Purification by flash chromatography on silica gel eluting with hexanes:ethyl acetate (20: 1) afforded 127 as an oil (1.74 g, 80%): ¹H NMR (CDCl₃, 400 MHz) δ 8.15 (m, 1 H), 7.42 (m, 1 H), 6.53 (dd, *J* = 6.8 and 4.8 Hz, 1 H), 6.48 (d, *J* =8.4 Hz, 1 H), 5.89-5.79 (m, 1 H), 5.14 (m, 1 H), 4.14 (d, *J=* 5.1 Hz, 2 H), 3.03 (s, 3 H); CIMS *m*/*z* 149 (M + H)⁺.

### 5-Methyl-2-phenyl-4-prop-2-enyloxazole (126).

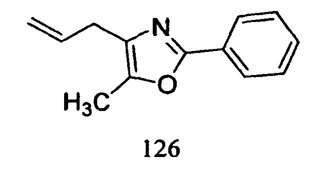

Amide 125 (2.00 g, 9.205 mmol) was dissolved in TFA (16 mL) and TFAA (8 mL) was added. The mixture was heated at 35°C-40°C for 16 hours. The mixture was allowed to cool and the solvents removed under reduced pressure. The residue was diluted with saturated NaHCO₃ (50 mL) and solid NaHCO₃ was added to neutralize the mixture. It then was extracted with ethyl acetate (3 × 60 mL). The combined organic extracts were washed with brine, dried over MgSO₄, filtered and the solvent removed. Purification by flash chromatography on silica gel eluting with hexanes:ethyl acetate (15:1) gave 135 (1.751 g, 95.5%): ¹H NMR (CDCl₃, 400 MHz) δ 7.98 (d, *J*= 7.8 Hz, 2 H), 7.43-7.35 (m, 3 H), 6.03-5.93 (m, 1H), 5.13 (dq, *J=* 16.9 and 1.7 Hz, 1 H), 5.09 (dq, *J=* 10.0 and 1.5 Hz, 1 H), 3.29 (d, J= 6.3 Hz, 2 H), 2.32 (s, 3 H); CIMS *m*/*z* 200 (M + H)⁺; IR 3070, 2924, 1638, 1450cm⁻¹.

### N-(1-Acetylbut-3-enyl)benzamide (125).

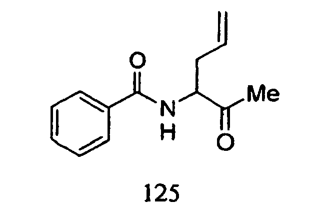

Benzamidoacetone (See Example 49) (2.098 g, 11.839 mmol) was dissolved in THF (120 mL) and cooled to -78°C under nitrogen. A 1.0 M solution of LHMDS in THF (11.9 mL, 11.9 mmol) was added and the mixture stirred for 40 minutes. A solution of allyl bromide (1.33 mL, 15.39 mmol) in THF (10 mL) was added. The mixture was allowed to warm to room temperature and stirred overnight. The mixture was diluted with brine and the phases were separated. The aqueous phase was extracted with ethyl acetate (3 × 50 mL) and the combined organic extracts were dried over MgSO₄, filtered, and the solvent removed. Purification by flash chromatography on silica gel eluting with hexanes:ethyl acetate (2:1 to 1:1) gave amide 9 (2.019 g, 78.5%): ¹H NMR (CDCl₃, 400 MHz) δ 7.79 (d, *J* = 7.1 Hz, 2 H), 7.51-7.41 (m, 3 H), 6.95 (bd, *J* = 5.4 Hz, 1 H), 5.74-5.64 (m, 1 H), 5.17-5.12 (m, 2 H), 4.88 (dt, *J=* 6.8 and 5.4 Hz, 1 H), 2.85-2.78 (m, 1 H), 2.61-2.54 (m, 1 H), 2.28 (s, 3 H); CIMS *m*/*z* 218 (M + H)⁺; IR 3263, 3081, 1719, 1632, 1556 cm⁻¹. Anal. calcd for C₁₃H₁₅NO₂: C, 71.87; H, 6.96; N, 6.45. Found: C, 71.91; H, 7.03; N, 6.52.

### (a-2) Esters 128c-d were prepared in the following manner.

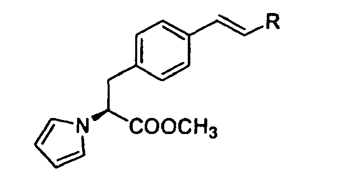

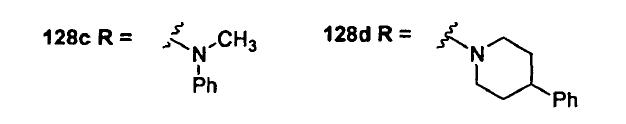

(*S*)-3-{4-[(*E*)-3-(Methyl-phenyl-amino)-propenyl]-phenyl}-2-pyrrol-1-yl-propionic acid methyl ester (128c). Triflate 127 (0.98 g, 2.592 mmol), boronate ester 124 (0.850 g, 3.111 mmol), K₂CO₃ (0.716 g, 5.184 mmol) were stirred in dry toluene (25 mL). Nitrogen was passed through the mixture for 0.5 hr. Pd(PPh₃)₄ (0.149 g, 0.129 mmol) was added and the reaction mixture was heated at 85°C-90°C for 24 hours. The mixture was allowed to cool, diluted with ethyl acetate (70 mL) and washed with sat NaHCO₃, water and brine. The organic extracts were dried over MgSO₄, filtered and the solvent removed. Purification by flash chromatography on silica gel eluting with 50-0% petroleum ether in dichloromethane followed by a second chromatographic purification eluting with hexanes:ethyl acetate (8:1 to 5:1) gave ester 137c as a 94:6 E:Z mixture of isomers (0.819 g, 84%). ***E*-isomer**: ¹H NMR (CDCl₃, 400 MHz) δ 7.23-7.20 (m, 2 H), 7.21 (d, *J*= 8.0 Hz, 2 H), 6.92 (d, *J* = 8.0 Hz, 2 H), 6.77-6.70 (m, 3 H), 6.69 (t, *J*= 2.0 Hz, 2 H), 6.44 (d, *J*= 16.0 Hz, 1H), 6.19 (dt, *J*= 15.6 and 5.6 Hz, 1 H), 6.13 (t, *J* = 2.0 Hz, 2 H), 4.71 (dd, *J* = 9.2 and 6.4 Hz, 1 H), 4.05 (dd, *J* = 5.6 and 1.2 Hz, 2 H), 3.69 (s, 3 H), 3.40-3.19 (m, 2 H), 2.95 (s, 3 H); CIMS *m*/*z* 375 (M + H)⁺.

(*S*)-3-{4-[(*E*)-3-(4-Phenyl-piperidin-1-yl)-propenyl]-phenyl}-2-pyrrol-1-yl-propionic acid methyl ester (128d). This compound was prepared from triflate 127 (2.0 g, 5.296 mmol), and boronate ester 124 (2.6 g, 7.944 mmol) following the procedure described for 128c. Purification by flash chromatography eluting with 33-45% ethyl acetate in hexanes containing 1% Et₃N gave 128d (E-isomer, 1.801 g, 79%) and E:Z mixture (0.217 g, 9.9%) as oils. ***E*-isomer**: ¹H NMR (CDCl₃, 400 MHz) δ 7.50-7.15 (m, 7 H), 6.95 (d, *J* = 8.4 Hz, 2 H), 6.69 (t, *J* = 2.0 Hz, 2 H), 6.45 (d, *J* = 16.0 Hz, 1 H), 6.27 (dt, *J* = 15.6 and 6.8 Hz, 1 H), 6.13 (t, *J* = 2.0 Hz, 2 H), 4.71 (dd, *J* = 8.8 and 6.4 Hz, 1 H), 3.68 (s, 3 H), 3.40-3.19 (m, 2 H), 3.16 (d, *J =* 6.8 Hz, 2 H), 3.08 (d, *J* = 11.6 Hz, 2 H), 2.51-2.46 (m, 1 H), 2.07 (t, *J* = 11.2 Hz, 1 H), 2.06 (t, *J* = 11.2 Hz, 1 H), 1.84-1.77 (m, 4 H); CIMS *m*/*z* 429 (M + H)⁺.

### Methyl-phenyl-[2-(4,4,5,5-tetramethyl-[1,3,2]dioxaborolan-2-yl)-vinyl]-amine (124).

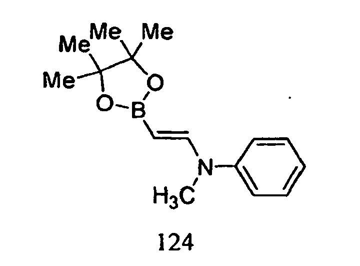

A mixture of *N*-methyl-*N*-prop-2-ynyl aniline (1.0 mL, 6.873 mmol) and pinacolborane (1.2 mL, 8.247 mmol) in CH₂Cl₂ (5 mL) was added to Cp₂ZrHCl (0.088 g, 0.343 mmol) at 0°C. The mixture was allowed to warm at room temperature and stirred for 8 days under nitrogen. At this time, the mixture was diluted with Et₂O (50 mL) and washed with water (30 mL). The phases were separated and the aqueous phase was extracted with Et₂O (2 × 30 mL). The combined organic extracts were dried over MgSO₄, filtered and the solvent removed. Purification by chromatography on silica gel eluting with hexanes: ethyl acetate (20:1 to 10:1) afforded 124 as an off-white solid (0.99 g, 53%): ¹H NMR (CDCl₃, 400 MHz) δ 7.21-7.17 (m, 2 H), 6.69-6.64 (m, 3 H), 6.60 (dt, *J =* 18.0 and 4.4 Hz, 1H), 5.55 (dt, *J =* 18.0 and 1.6 Hz, 1 H), 3.99 (dd, *J =* 4.4 and 1.6 Hz, 2 H), 2.94 (s, 3 H), 1.25 (s, 12 H); CIMS *m*/*z* 274 (M + H)⁺.

(*S*)-3-{4-[(*E*)-3-(Methyl-phenyl-amino)-propenyl]-phenyl}-2-pyrrol-1-yl-propionic acid methyl ester (123). This compound was prepared from triflate 119 (2.0 g, 5.296 mmol), and boronate ester 124 (2.6 g, 7.944 mmol) following the procedure described for 137c. Purification by flash chromatography eluting with 33-45% ethyl acetate in hexanes containing 1% Et₃N gave 123 (*E*-isomer, 1.801 g, 79%) and E:Z mixture (0.217 g, 9.9%) as oils. ***E*-isomer:** ¹H NMR (CDCl₃, 400 MHz) δ 7.50-7.15 (m, 7 H), 6.95 (d, *J* = 8.4 Hz, 2 H), 6.69 (t, *J =* 2.0 Hz, 2 H), 6.45 (d, *J*= 16.0 Hz, I H), 6.27 (dt, *J*= 15.6 and 6.8 Hz, 1 H), 6.13 (t, *J*= 2.0 Hz, 2 H), 4.71 (dd, *J* = 8.8 and 6.4 Hz, 1 H), 3.68 (s, 3 H), 3.40-3.19 (m, 2 H), 3.16 (d, J= 6.8 Hz, 2 H), 3.08 (d, *J* = 11.6 Hz, 2 H), 2.51-2.46 (m, 1 H), 2.07 (t, *J*=11.2 Hz, 1 H), 2.06 (t, *J*= 11.2 Hz, 1 H), 1.84-1.77 (m, 4 H); CIMS *m*/*z* 429 (M + H)⁺.

### 4-Phenyl-1-[2-(4,4,5,5-tetramethyl-[1,3,2]dioxaborolan-2-yl)-vinyl]-piperidine(130).

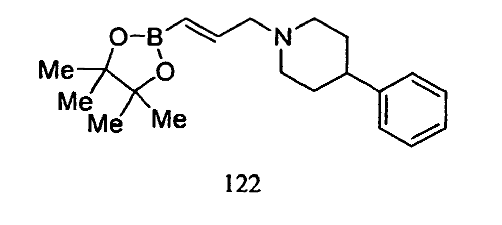

A mixture of *N*-prop-2-ynyl-4-phenylpiperidine (1.5 g, 7.526 mmol) and pinacolborane (1.64 mL, 11.289 mmol) in CH₂Cl₂ (8 mL) was added to Cp₂ZrHCl (0.194 g, 0.753 mmol) at 0°C. The mixture was allowed to warm at room temperature and stirred for 48 hours under nitrogen. At this time, the mixture was diluted with Et₂O (50 mL) and water (30 mL) was added carefully. The phases were separated and the organic extracts were dried over MgSO₄, filtered and the solvent removed to give 18 as a solid (2.46 g, 100%): ¹H NMR (CDCl₃, 400 MHz) δ 7.29-7.18 (m, 5 H), 6.68 (dt, *J*= 17.6 and 6.0 Hz, 1 H), 5.62 (dt, *J*= 18.0 and 1.6 Hz, 1 H), 3.11 (dd, *J* = 6.4 and 1.6 Hz, 2H), 3.04 (d, *J* = 12.0 Hz, 2 H), 2.51-2.44 (m, 1H), 2.10-2.03 (m, 2 H), 1.84-1.79 (m, 4 H), 1.26 (s, 12 H); CIMS *m*/*z* 328 (M + H)⁺.

### Example 5

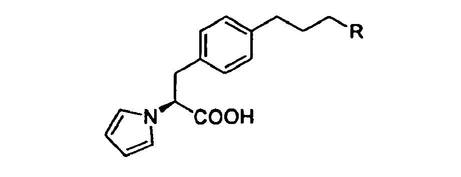

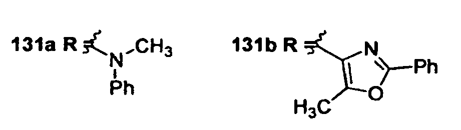

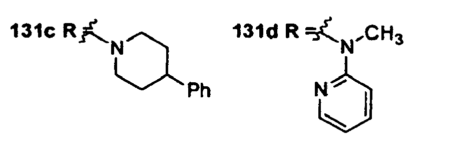

### General procedure for the preparation of analogs 131a-d.

(*S*)-3-{4-[3-(Methyl-phenyl-amino)-propyl]-phenyl}-2-pyrrol-1-yl-propionic acid (131a). Ester 130a (0.223 g, 0.592 mmol) was dissolved in THF:H₂O (10:4 mL) and LiOH·H₂O (0.037 g, 0.888 mmol) was added and the mixture was stirred at room temperature for 3.5 hours. The solvent was removed and the residue was diluted with water, acidified with 10% HCl. The mixture was extracted with CHCl₃ (3 × 50 mL). The combined organic extracts were dried over MgSO₄, filtered and the solvent removed. Purification by chromatography on silica gel eluting with 0-5% MeOH in CHCl₃ containing formic acid (0-0.1 %) gave pure 140a as pale brown solid (0.190 g, 88%): ¹H NMR (CDCl₃, 400 MHz) δ 7.33 (m, 2 H), 7.12-7.06 (m, 3 H), 6.95 (d, *J* = 8.3 Hz, 2 H), 6.91 (d, *J* = 8.3 Hz, 2 H), 6.69 (t, *J* = 2.0 Hz, 2 H), 6.10 (t, *J* = 2.0 Hz, 2 H), 4.73 (dd, *J* = 9.1 and 6.3 Hz, 1 H), 3.42-3.18 (m, 4 H), 2.52 (t, *J* = 7.3 Hz, 2 H), 1.88-1.81 (m, 2 H). Anal. calcd for C₂₃H₂₆N₂O₂·0.1 H₂O: C, 75.84; H, 7.25; N, 7.69. Found: C, 75.73; H, 7.40; N, 7.50.

(*S*)-3-{4-[3-(5-Methyl-2-phenyl-oxazol-4-yl)-propyl]-phenyl}-2-pyrrol-1-yl-propionic acid (131b). Prepared from ester 130b (0.243 g, 0.567 mmol) following the general procedure. Purification by flash chromatography eluting with 33-50% ethyl acetate in hexanes containing formic acid (0-0.2%) gave pure 140b as a light yellow solid (0.206 g, 88%): mp 167°C-168°C; ¹H NMR (CDCl₃, 400 MHz) δ 7.94 (m, 2 H), 7.40 (m, 3 H), 7.04 (d, *J* = 7.8 Hz, 2 H), 6.93 (d, *J* = 7.8 Hz, 2 H), 6.71 (bs, 2 H), 6.14 (bs, 2 H), 4.72 (m, 1 H), 3.39-3.16 (m, 2 H), 2.58 (t, *J*= 7.3 Hz, 2 H), 2.47 (t, *J*= 7.3 Hz, 2 H), 2.26 (s, 3 H), 1.92 (qn, *J*= 7.3 Hz, 2 H); CIMS *m*/*z* 415 (M + H)⁺.

(*S*)-3-{4-[3-(4-Phenyl-piperidin-1-yl)-propyl]-phenyl}-2-pyrrol-1-yl-propionic acid (131c). Prepared from ester 131c (0.248 g, 0.576 mmol) by the general procedure described above. Purification by chromatography on silica gel eluting with 0-9% MeOH in CHCl₃ containing formic acid (0-0.1%) gave pure 139c as a white foam (0.137 g, 57%): mp 95°C-100°C; ¹H NMR (CDCl₃, 400 MHz) δ 7.29-7.16 (m, 3 H), 7.14 (d, *J* = 8.3 Hz, 2 H), 6.98 (d, *J* = 8.3 Hz, 2 H), 6.95 (d, *J* = 8.0 Hz, 2 H), 6.67 (t, *J* = 2.0 Hz, 2 H), 6.04 (t, *J* = 2.0 Hz, 2 H), 4.64 (t, *J* = 7.5 Hz, 1 H), 3.50 (m, 1 H), 3.45 (m, 1 H), 3.41-3.09 (m, 2 H), 2.82-2.78 (m, 2 H), 2.57-2.33 (m, 4 H), 2.21 (m, 2 H), 2.03-1.99 (m, 2 H), 1.84 (m, 2 H); CIMS *m*/*z* 417 (M + H)⁺. Anal. calcd for C₂₇H₃₂N₂O₂·1.0 H₂O: C, 74.62; H, 7.89; N, 6.45. Found: C, 74.23; H, 7.63; N, 6.25.

(*S*)-3-(4-[3-(Methyl-pyridin-2-yl-amino)-propyl]-phenyl)-2-pyrrol-1-yl-propionic acid (131d). Prepared from ester 131d (0.378 g, 1.001 mmol) by the general procedure. Purification by flash chromatography eluting with 0-15% MeOH in CHCl₃ gave 142d as a white solid (0.280 g, 77%): mp 66°C-68°C; ¹H NMR (CD₃OD, 400 MHz) δ 7.90-7.85 (m, 1 H), 7.82 (d, *J=* 6.0 Hz, 1 H), 7.01 (t, *J* = 8.0 Hz, 3 H), 6.94 (d, *J* = 8.0 Hz, 2 H), 6.87 (t, *J =* 6.8 Hz, 1 H), 6.66 (t, *J =* 2.0 Hz, 2 H), 5.96 (t, *J =* 2.0 Hz, 2 H), 4.80 (dd, *J =* 10.0 and 5.6 Hz, 1H), 3.55 (t, *J =* 7.6 Hz, 2 H), 3.37-3.15 (m, 2 H), 3.12 (s, 3 H), 2.62 (t, *J =* 7.6 Hz, 2 H), 1.94 (qn, *J* = 7.2 Hz, 2 H); CIMS *m*/*z* 364 (M + H)⁺. Anal. calcd for C₂₂H₂₅N₃O₂·0.3 CHCl₃: C, 67.08; N, 6.39; N, 10.52. Found: C, 67.30; H, 6.39; N, 10.21.

(a) The esters 130a-c were prepared in the following manner.

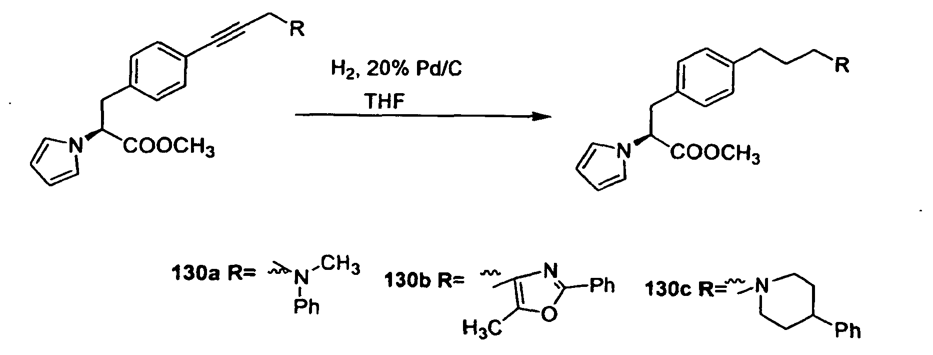

*(S)-*3-{4-[3-(Methyl-phenyl-amino)-propyl]-phenyl}-2-pyrrol-1-yl-propionic acid methyl ester (130a). Alkyne 120a (0.648 g, 1.739 mmol) was dissolved in MeOH (50 mL) and 20% Pd/C (0.050 g) was added. The mixture was hydrogenated at room temperature for 18 hr. The mixture was filtered through celite and the solvent removed. Purification by flash chromatography eluting with hexanes:ethyl acetate (6:1) gave 130a as an oil (0.439 g, 67%): ¹H NMR (CDCl₃, 400 MHz) δ 7.20 (t, *J*= 8.0 Hz, 2 H), 7.05 (d, J= 7.6 Hz, 2 H), 6.92 (d, J= 8.0 Hz, 2 H), 6.71 (t, *J =* 2.0 Hz, 2 H), 6.65 (t, *J* = 7.8 Hz, 3 H), 6.14 (t, *J =* 2.0 Hz, 2 H), 4.73 (dd, *J* = 8.8 and 6.4 Hz, 1H), 3.69 (s, 3 H), 3.41-3.20 (m, 2 H), 3.30 (t, *J* = 7.4 Hz, 2 H), 2.90 (s, 3 H), 2.59 (t, *J* = 7.6 Hz, 2 H), 1.86 (qn, *J* = 7.6 Hz, 2 H); CIMS *m*/*z* 377 (M + H)⁺.

(*S*)-3-{4-[3-(5-Methyl-2-phenyl-oxazol-4-yl)-propyl]-phenyl}-2-pyrrol-1-yl-propionic acid methyl ester (130b). This compound was prepared from alkyne 120b (0.249 g, 0.586 mmol) following the general procedure described for 130a with the exception that the reaction was carried out in THF. The crude product was used for subsequent transformations. Ester 130b was obtained as an oil (0.243, 96.8%): ¹H NMR (CDCl₃, 400 MHz) δ 7.91 (dd, *J =* 8.0 and 1.6 Hz, 2 H), 7.37-7.32 (m, 3 H), 6.99 (d, *J* = 8.4 Hz, 2 H), 6.85 (d, *J* = 8.0 Hz, 2 H), 6.64 (t, *J =* 2.0 Hz, 2 H), 6.07 (t, *J =* 2.0 Hz, 2 H), 4.65 (dd, *J* = 8.8 and 6.8 Hz, 1 H), 3.62 (s, 3 H), 3.33-3.11 (m, 2 H), 2.54 (t, *J* = 7.6 Hz, 2 H), 2.41 (t, *J =* 7.6 Hz, 2 H), 2.19 (s, 3 H), 1.89 (qn, *J* = 7.6 Hz, 2 H); CIMS *m*/*z* 429 (M + H)⁺.

(S)-3-{ 4-[3-(4-Phenyl-piperidin-1-yl)-propyl]-phenyl}-2-pyrrol-1-yl-propionic acid methyl ester (130c). This compound was prepared from alkyne 120c (0.408 g, 0.956 mmol) following the procedure described for 130a. The reaction was carried out in THF. Purification was achieved by chromatography eluting with 33-45% ethyl acetate in hexanes containing 1.0% Et₃N to give 130c as an oil (0.262 g, 64%): ¹H NMR (CDCl₃, 400 MHz) δ 7.24-7.10 (m, 5 H), 7.00 (d, *J* = 8.0 Hz, 2 H), 6.85 (d, *J* = 8.0 Hz, 2 H), 6.65 (t, *J*= 2.0 Hz, 2 H), 6.08 (t, *J* = 2.0 Hz, 2 H), 4.66 (dd, J= 8.8 and 6.4 Hz, 1 H), 3.62 (s, 3 H), 3.34-3.12 (m, 2 H), 2,98 (d, *J* = 11.6 Hz, 2 H), 2.52 (t, *J =* 7.6 Hz, 2 H), 2.42 (m, 1 H), 2.31 (t, *J =* 7.6 Hz, 2 H), 1.99-1.93 (m, 2 H), 1.80-1.72 (m, 6 H); CIMS *m*/*z* 431 (M + H)⁺.

### (S)-3-{4-[3-(Methyl-pyridin-2-yl-amino)-propyl]-phenyl}-2-pyrrol-1-yl-propionic acid methyl ester (130d).

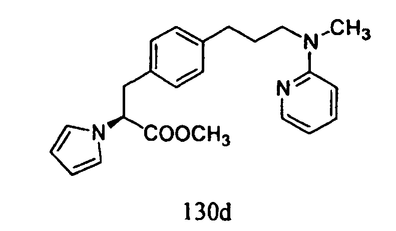

Alkene 128b (0.430 g, 1.145 mmol) was dissolved in THF (16 mL). 5% Pd/C (0.050 g) was added and the mixture was hydrogenated at room temperature for 24 hours. Additional catalyst (0.050 g) was added and the reaction was continued for another 24 hours. The catalyst was filtered off and the solvent removed to give ester 130d as a thick oil (0.378 g, 87%): ¹H NMR (CDCl₃, 400 MHz) δ 8.08-8.06 (m, 1 H), 7.36-7.31 (m, 1 H), 6.99 (d, *J* = 8.0 Hz, 2 H), 6.85 (d, *J* = 8.0 Hz, 2 H), 6.64 (t, J= 2.0 Hz, 2 H), 6.43 (dd, *J=* 6.4 and 5.4 Hz, 1 H), 6.33 (d, *J* = 8.8 Hz, 6.07 (t, *J* = 2.0 Hz, 2 H), 6.65 (dd, *J* = 8.8 and 6.8 Hz, 1 H), 3.62 (s, 3 H), 3.45 (t, *J*= 7.4 Hz, 2 H), 3.33-3.12 (m, 2 H), 2.94 (s, 3 H), 2.53 (t, *J*= 7.6 Hz, 2 H), 1.81 (qn, *J*= 7.6 Hz, 2 H); CIMS *m*/*z* 378 (M + H)⁺.

### Example 6

### 3-{4-[3-(5-Methyl-2-phenyl-oxazol-4-yl)-propyl]-phenyl}-2-1,2,3-triazol-1-yl-propionic acid (242).

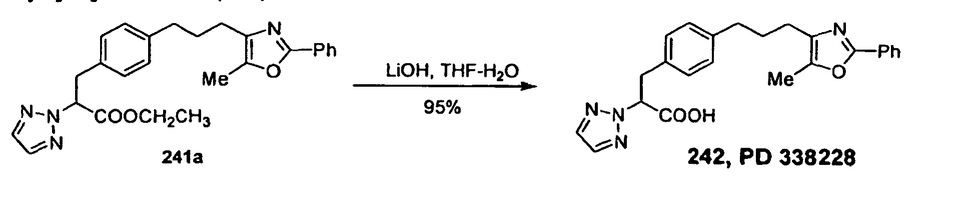

GENERAL METHOD 91. 3-{4-[3-(5-Methyl-2-phenyl-oxazol-4-yl)-propyl]-phenyl}-2-1,2,3-triazol-2-yl-propionic acid ethyl ester (241a) (2.37 g, 5.331 mmol) was dissolved in THF (60 mL) and water was added (15 mL). Lithium hydroxide monohydrate (0.335 g, 8.00 mmol) was incorporated and the mixture was stirred at room temperature for 1 h. The organic solvent was removed in the rotary evaporator at room temperature. The aqueous residue was diluted with water and extracted with Et₂O (2 × 45 mL). A flow of air was passed through the aqueous phase to remove residual ether. The aqueous phase was then acidified with 10% HCl. The precipitated solid was separated by vacuum filtration and washed with water. The solid was dried on air overnight and then at 45 °C for 14 h to give the title compound as an off-white solid (2.11 g, 95%): mp (softens or melts between 50-85 °C, forms another solid that melts at 153-153.5 °C); ¹H NMR (CDCl₃, 400 MHz) δ 7.98-7.96 (m, 2 H), 7.62 (s, 2 H), 7.43-7.40 (m, 3 H), 7.03 (d, *J* = 8.1 Hz, 2 H), 6.98 (d, *J* = 8.1 Hz, 2 H), 5.52 (dd, J= 10.4 and 5.0 Hz, 1 H), 3.65-3.49 (m, 2 H), 2.58 (t, *J* = 7.6 Hz, 2 H), 2.48 (t, *J=* 7.6 Hz, 2 H), 2.27 (s, 3 H), 1.93 (m, *J =* 7.6 Hz, 2 H); CIMS *mlz* 417 (M+1). HPLC: purity: 100%; column: symmetry C₁₈, 4.6 × 150 mm, 5µM; mobile phase: A: water +0.1% TFA, B: acetonitrile +0.1% TFA; retention time: 15.914 min; wavelength: 254 nm.

3-{4-[3-(5-Methyl-2-phenyl-oxazol-4-yl)-propyl]-phenyl}-2-1,2,3-triazol-2-yl-propionic acid ethyl ester (241a) was prepared in the following manner.

### (a) 3-{4-[3-(5-Methyl-2-phenyl-oxazol-4-yl)-propyl]-phenyl}-2-1,2,3-triazol-2-yl-propionic acid ethyl ester (241a).

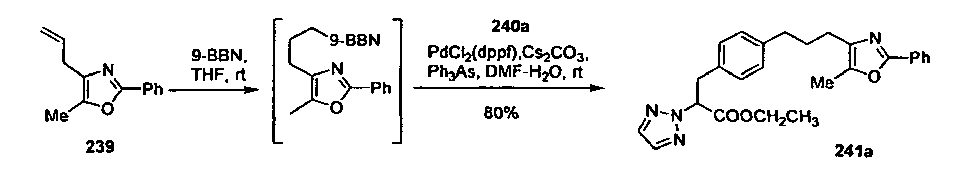

GENERAL METHOD 92-a. A solution of 5-methyl-2-phenyl-4-prop-2-enyloxazole (compound 239) (1.716 g, 8.614 mmol) in dry THF (25 mL) was added to a 0.5 M solution of 9-BBN in THF (34.45 mL) at 0 °C under a nitrogen atmosphere. The ice bath was removed and the mixture was stirred at room temperature overnight. The 9-BBN adduct was then added to a flask containing bromide 238a (2.148 g, 6.626 mmol), PdCl₂(dppf) (0.485 g, 0.662 mmol), Cs₂CO₃ (3.88 g, 11.927 mmol), Ph₃As (0.203 g, 0.662 mmol), water (1.4 mL, 79.5 mmol) and DMF (15 mL). The reaction mixture was stirred at room temperature under a nitrogen atmosphere for 2 days. At the end of this time, the mixture was cooled in an ice bath and 3 M NaOAc (16 mL) was added followed by 30% H₂O₂ (8 mL). Stirring was continued for 2 h, allowing the reaction to warm to room temperature slowly. Water (100 mL) was added followed by Et₂O and EtOAc. The phases were separated and the aqueous phase was extracted with Et₂O-EtOAc (60:10 mL × 4). The combined organic extracts were washed with brine, dried over magnesium sulfate and the solvent removed. Purification by column chromatography on silica gel eluting with EtOAc in hexanes (0 to 22%) afforded ester 241a as a thick oil (2.37 g, 80%): ¹H NMR (CDCl₃, 400 MHz) δ 7.98-7.95 (m, 2 H), 7.61 (s, 2 H), 7.44-7.38 (m, 3 H), 7.03 (d, *J*= 8.3 Hz, 2 H), 7.00 (d, *J*= 8.3 Hz, 2 H), 5.50 (dd, *J =* 10.2 and 5.6 Hz, 1 H), 4.18 (q, *J =* 7.1 Hz, 2 H), 3.71-3.57 (m, 2 H), 2.58 (t, J= 7.6 Hz, 2 H), 2.46 (t, *J =* 7.6 Hz, 2 H), 2.24 (s, 3 H), 1.93 (m, *J*= 7.6 Hz, 2 H), 1.19 (t, *J*= 7.1 Hz, 3 H); CIMS *m*/*z* 445 (M+1).

### (b) Bromide 240a (PD 0341554).

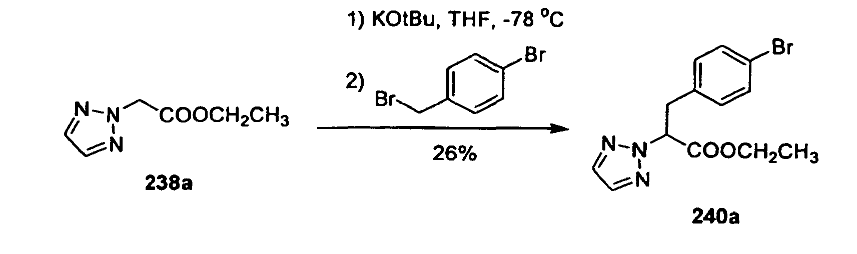

General Method 91-b. Ester 238a (3.0 g, 19.336 mmol) was dissolved in dry THF (60 mL) and cooled to -78°C under a nitrogen atmosphere. A 1.0 M solution of potassium *tert*-butoxide in THF (20.3 mL, 20.3 mmol) was added. The mixture was stirred at -78 °C for 45 min. A solution of 4-bromobenzyl bromide (5.56 g, 22.236 mmol) in THF (20 mL) was added. The reaction was allowed to reach room temperature slowly and then stirred at room temperature for 6 days. The mixture was quenched with water (60 mL) and diluted with Et₂O and EtOAc. The phases were separated and the aqueous phase was extracted with Et₂O-EtOAc (50:5 mL × 3). The combined organic extracts were washed with brine, dried over magnesium sulfate and the solvent removed. Purification by column chromatography on silica gel eluting with EtOAc in hexanes (0 to 16%) afforded the title compound as an oil (1.668 g, 26%): ¹H NMR (CDCl₃, 400 MHz) δ 7.62 (s, 2 H), 7.32 (d, *J* = 8.5 Hz, 2 H), 6.97 (d, *J* = 8.5 Hz, 2 H), 5.48 (dd, *J* = 10.4 and 5.4 Hz, 1 H), 4.19 (q, *J*= 7.1 Hz, 2 H), 3.70-3.55 (m, 2 H), 1.20 (t, *J*= 7.1 Hz, 3 H); CIMS *m*/*z* 324 (M)⁺.

### (c) 2-(2H-1,2,3-Triazol-2-yl)ethyl acetate (238a).

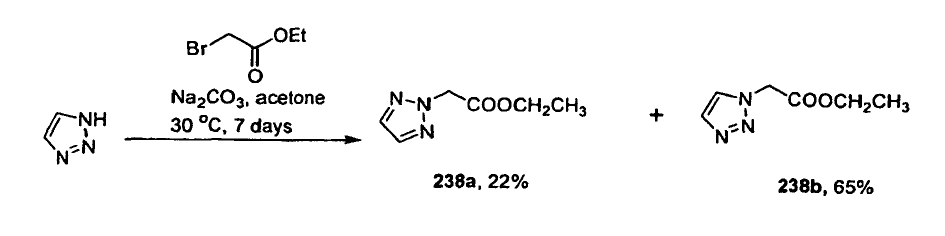

The title compound was prepared following the procedure described by Kume et. al.( Kume, M.; Kubota, T.; Kimura, Y.; Nakashimizu, H.; Motokawa, K.; Nakano, M. *J Antibiotics* **1993**, *46*,177-192.) Following this procedure, starting from 1*H*-1,2,3-triazole (18.17 g, 0.263 mol), ester 238a was obtained as a liquid (9.2 g, 22%) after column chromatography on silica gel eluting with EtOAc in hexanes (0 to 55%): ¹H NMR (CDCl₃, 400 MHz) δ 7.68 (s, 2 H), 5.23 (s, 2 H), 4.24 (q, *J*= 7.1 Hz, 2 H), 1.27 (t, *J=* 7.1 Hz, 3 H); CIMS *m*/*z* 156 (M+1).

From the same purification, the *N*-1 alkylated product 238b was obtained as the major product (26.45 g, 65%): ¹H NMR (CDCl₃, 400 MHz) δ 7.75 (d, *J*= 1.0 Hz, 1 H), 7.71 (d, *J* = 1.0 Hz, 1 H), 5.19 (s, 2 H), 4.25 (q, *J* = 7.1 Hz, 2 H), 1.28 (t, *J=* 7.1 Hz, 3 H); CIMS *mlz* 156 (M+1).

### Example 7

### 3-{4-[3-(5-Methyl-2-phenyl-oxazol-4-yl)-propyl]-phenyl}-2-1,2,3-triazol-1-yl-propionic acid (245).

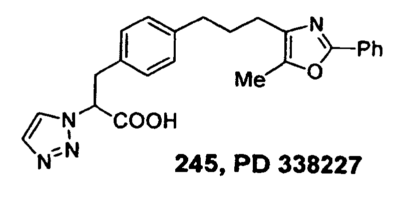

Prepared from ester 244 (1.0 g, 2.249 mmol) following General Method 92. The title compound was obtained as white solid (0.854 g, 91%): mp 162-163.5 °C; ¹H NMR (CDCl₃, 400 MHz) δ 7.93 (m, 2 H), 7.66 (s, 1 H), 7.63 (s, 1 H), 7.41 (m, 3 H), 7.01 (d, J = 8.0 Hz, 2 H), 6.92 (d, J = 8.0 Hz, 2 H), 5.62 (dd, J = 8.3 and 6.1 Hz, 1 H), 3.46-3.35 (m, 2 H), 2.56 (t, J = 7.3 Hz, 2 H), 2.48 (t, J = 7.3 Hz, 2 H), 2.28 (s, 3 H), 1.89 (m, 2 H); CIMS *mlz* 417 (M+1).

3-{4-[3-(5-Methyl-2-phenyl-oxazol-4-yl)-propyl]-phenyl}-2-1,2,3-triazol-1-yl-propionic acid methyl ester (244) was prepared in the following manner.

### (a) 3-{4-[3-(5-Methyl-2-phenyl-oxazol-4-yl)-propyl]-phenyl}-2-1,2,3-triazol-1-yl-propionic acid methyl ester (244).

The title compound was prepared from bromide 243 (1.2 g, 3.702 mmol) using the General Method 92a. Ester 244b was obtained as a thick oil (1.00 g, 61 %): ¹H NMR (CDCl₃, 400 MHz) δ 7.98 (m, 2 H), 7.66 (s, 1H), 7.63 (s, I H), 7.44-7.38 (m, 3 H), 7.06 (d, J = 8.0 Hz, 2 H), 6.93 (d, J = 8.0 Hz, 2 H), 5.59 (dd, J = 8.3 and 6.6 Hz, 1H), 4.19 (q, J = 7.3 Hz, 2 H), 3.49-3.38 (m, 2 H), 2.61 (t, J = 7.6 Hz, 2 H), 2.48 (t, J = 7.3 Hz, 2 H), 2.27 (s, 3 H), 1.96 (m, 2 H), 1.21 (t, J = 7.1 Hz, 3 H); CIMS *m*/*z* 445 (M+1).

### (b) Bromide 243

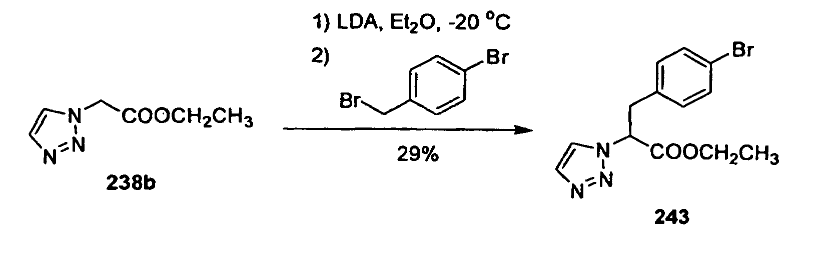

A solution of diisopropylamine (2.0 mL, 14.18 mmol) in Et₂O (30 mL) was cooled at -20 °C under a nitrogen atmosphere. A 1.6 M solution of BuLi in hexanes (9.7 mL, 15.47 mmol) was added. The mixture was stirred for 25 min. A solution of ester 238b (2.0 g, 12.89 mmol) in Et₂O (20 mL) was added. The mixture was stirred for 30 min. A solution of 4-bromobenzylbromide (3.70 g, 14.82 mmol) in Et₂O (20 mL) was incorporated. The reaction mixture was kept at -20 °C for 2.5 h and then allowed to warm to room temperature slowly. Stirring was continued at room temperature for 24 h. At this time, the mixture was quenched with water and diluted with Et₂O. The phases were separated and the aqueous layer was extracted with Et₂O (3 × 40 mL). The combined organic extracts were washed with brine, dried over magnesium sulfate and the solvent removed. Purification by column chromatography over silica gel afforded bromide 243 as a pale yellow oil (1.21 g, 29%): ¹H NMR (CDCl₃, 400 MHz) δ 7.67 (d, J = 0.7 Hz, 1 H), 7.63 (d, J = 1.0 Hz, 1 H), 7.35 (d, J = 8.5 Hz, 2 H), 6.89 (d, J = 8.5 Hz, 2 H), 5.54 (dd, J = 8.7 and 6.5 Hz, 1 H), 4.21 (q, J = 7.1 Hz, 2 H), 3.51-3.39 (m, 2 H), 1.22 (t, J = 7.3 Hz, 3 H); CIMS *m*/*z* 324 (M)⁺.

### Example 8

### 3-{4-[3-(5-Methyl-2-phenyl-oxazol-4-yl)-propyl]-phenyl}-2-pyrazol-1-yl-propionic acid (249).

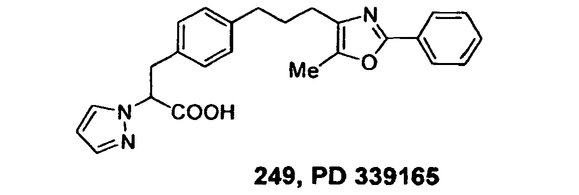

The title compound was prepared from 248 (0.735 g, 1.657 mmol) by the General Method 92. PD 0339165 was obtained as a yellowish solid (0.602 g, 87%): mp 75-77 °C; ¹H NMR (CDCl₃, 400 MHz) δ 7.96 (m, 2 H), 7.61 (d, J = 2.0 Hz, 1 H), 7.40 (m, 3 H), 7.09 (d, J = 2.0 Hz, 1 H), 7.02 (d, J = 8.0 Hz, 2 H), 6.80 (d, J = 8.0 Hz, 2 H), 6.19 (t, J = 2.2 Hz, 1H), 5.02 (dd, J = 10.0 and 4.6 Hz, 1 H), 3.45-3.27 (m, 2 H), 2.59 (t, J = 7.6 Hz, 2 H), 2.47 (t, J = 7.6 Hz, 2 H), 2.27 (s, 3 H), 1.93 (m, 2 H); CIMS *m*/*z* 416 (M+1). Anal. calcd for C₂₅H₂₅N₃O₃·0.4 H₂O: C, 71.04; H, 6.15; N, 9.94. Found: C, 70.76; H, 6.08; N, 9.91.

3-{4-[3-(5-Methyl-2-phenyl-oxazol-4-yl)-propyl]-phenyl}-2-pyrazol-1-yl-propionic acid ethyl ester (248) was prepared in the following manner.

### (a) 3-{4-[3-(5-Methyl-2-phenyl-oxazol-4-yl)-propyl]-phenyl}-2-pyrazol-1-yl-propionic acid ethyl ester (4c).

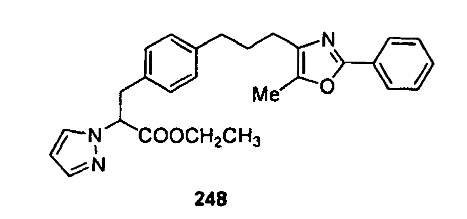

Prepared from bromide 247 (0.760 g, 2.351 mmol) by the General Method 95. Purification by column chromatography afforded 248 (0.735 g, 70%): ¹H NMR (CDCl₃, 400 MHz) δ 7.96 (m, 2 H), 7.52 (d, J = 1.5 Hz, 1 H), 7.44-7.38 (m, 4 H), 7.05 (d, J = 8.0 Hz, 2 H), 6.94 (d, J = 8.0 Hz, 2 H), 6.22 (t, J = 2.1 Hz, 1 H), 5.11 (t,J=7.7Hz, 1 H),4.16(q,J=7.1 Hz, 2 H), 2.60 (t, J = 7.6 Hz, 2 H), 2.47 (t, J = 7.3 Hz, 2 H), 2.26 (s, 3 H), 1.95 (m, 2 H), 1.18 (t, J = 7.1 Hz, 3 H); CIMS *m*/*z* 444 (M+1).

### (b) Pyrazol-1-yl acetic acid ethyl ester (247).

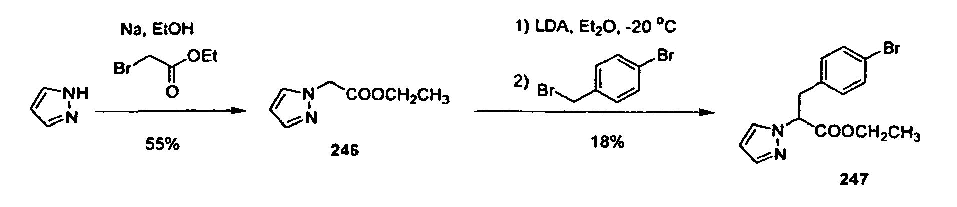

Sodium (3.7 g, 161 mmol) was dissolved in ethanol (150 mL) with ice cooling. Pyrazole (10 g, 146 mmol) was added. Ethyl bromoacetate (32 mL, 292 mmol) was added dropwise. The ice bath was removed after the addition was finished and the mixture stirred at room temperature for 5 days. The solvent was removed and the residue diluted with cold 6 N HCl and extracted with Et₂O (2 × 50 mL). The aqueous layer was neutralized with solid sodium carbonate, then it was extracted with chloroform. The combined organic extracts were washed with brine, dried over magnesium sulfate and the solvent removed. Purification by distillation afforded 247 as an oil (12.495 g, 55%): bp 65-80 °C at 2 mm Hg; ¹H NMR (CDCl₃, 400 MHz) δ 7.55 (d, J = 1.7 Hz, 1H), 7.47 (d, J = 2.2 Hz, 1H), 6.32 (t, *J* = 2.0 Hz, 1 H), 4.92 (s, 2 H), 4.22 (q, J = 7.1 Hz, 2 H), 1.27 (t, J = 7.1 Hz, 3 H); CIMS *mlz* 155 (M+1).

**Bromide 246.** The title compound was prepared from ester 246 (2.0 g, 12.97 mmol) by General Method 92-b. Bromide 247 was obtained as a thick oil (0.768 g, 18%): ¹H NMR (CDCl₃, 400 MHz) δ 7.53 (d, *J* = 1.7 Hz, 1 H), 7.36 (d, *J* = 2.0 Hz, 1 H), 7.33 (d, *J* = 8.5 Hz, 2 H), 6.88 (d, *J* = 8.3 Hz, 2 H), 5.07-5.03 (m, 1 H), 4.18 (q, J= 7.0 Hz, 2 H), 3.49-3.39 (m, 2 H), 1.20 (t, *J* = 7.1 Hz, 3 H); CIMS *m*/*z* 323 (M)⁺.

### Example 9

### 3-{4-[3-(5-Methyl-2-phenyl-oxazol-4-yl)-propyl]-phenyl}-2-1,2,4-triazol-1-yl-propionic acid (253).

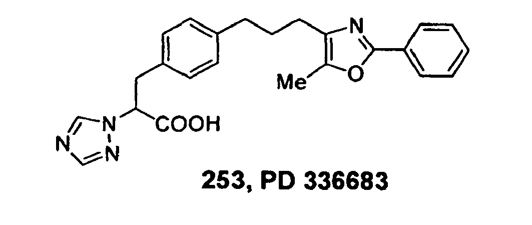

Prepared from ester 252 (0.492 g, 1.106 mmol) by the General Method 92. The title compound was obtained as a white solid (0.33 g, 72%): mp 169-171 °C; ¹H NMR (CDCl₃, 400 MHz) δ 8.16 (s, 1 H), 8.00 (m, 2 H), 7.98 (s, 1 H), 7.45-7.40 (m,3 H), 7.01 (d, J = 8.0 Hz, 2 H), 6.87 (d, J = 8.0 Hz, 2 H), 5.24 (t, J = 7.1 Hz, 1 H), 3.42 (d, J = 7.1 Hz, 2 H), 2.57 (t, J = 7.6 Hz, 2 H), 2.52 (t, J = 7.6 Hz, 2 H), 2.28 (s, 3 H), 1.93 (m, 2 H); CIMS *m*/*z* 417 (M+1).

3-{4-[3-(5-Methyl-2-phenyl-oxazol-4-yl)-propyl]-phenyl}-2- 1,2,4-triazol-1-yl-propionic acid ethyl ester (252) was prepared in the following manner.

### (a) 3-{4-[3-(5-Methyl-2-phenyl-oxazol-4-yl)-propyl]-phenyl}-2-1,2,4-triazol-1-yl-propionic acid ethyl ester (252).

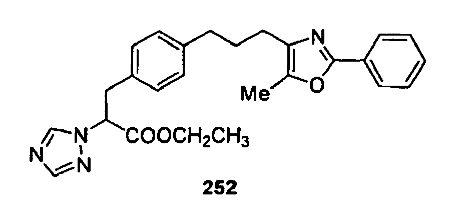

Prepared from bromide 251 (0.440 g, 1.357 mmol) by the General Method 95-a. The title compound was obtained as a thick oil (0.492 g, 82%): ¹H NMR (CDCl₃, 400 MHz) δ 8.08 (m, 2 H), 8.00 (s, 1 H), 7.94 (s, 1 H), 7.48-7.41 (m, 3 H), 7.06 (d, J = 8.0 Hz, 2 H), 6.88 (d, J = 8.0 Hz, 2 H), 5.17 (dd, J = 8.0 and 6.6 Hz, 1H), 4.21 (q, J = 7.1 Hz, 2 H), 3.43 (d, J = 7.3 Hz, 2 H), 2.62 (t, J = 7.6 Hz, 2 H), 2.53 (d, J = 7.3 Hz, 2 H), 2.28 (s, 3 H), 1.23 (t, J = 7.1 Hz, 3 H); CIMS *m*/*z* 445 (M+1).

### (b) Ethyl 2-1,2,4-Triazol-1-yl acetate (250) and Bromide (251).

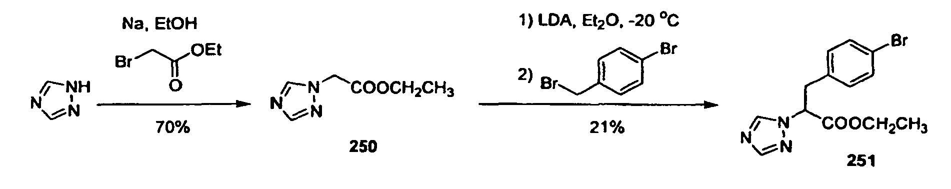

Ester 250 was prepared following the procedure described by Ainsworth and Jones (Ainsworth, C.; Jones, R. G. *J. Am. Chem. Soc.* **1955**, *77*, 621-624). ¹H NMR (CDCl₃, 400 MHz) δ 8.19 (s, 1H), 7.97 (s, 1H), 4.96 (s, 2 H), 4.25 (q, J = 7.1 Hz, 2 H), 1.28 (t, J =7.1 Hz, 3 H); CIMS *m*/*z* 156 (M+1).

**Bromide 251**. The title compound was prepared from ester 250 (1.0 g, 6.445 mmol) following the General Method III. Bromide 251 was obtained as a thick oil (0.447 g, 21%): ¹H NMR (CDCl₃, 400 MHz) δ 8.10 (s, 1 H), 7.98 (s, 1 H), 7.35 (d, J = 8.3 Hz, 2 H), 6.86 (d, J = 8.5 Hz, 2 H), 5.16 (dd, J = 8.1 and 6.7 Hz, 1H), 4.22 (q, J = 7.1 Hz, 2 H), 3.46 (d, J 7.6 Hz, 2 H), 1.23 (t, J = 7.1 Hz, 3 H); CIMS *mlz* 324 (M)⁺.

### Example 10

### 5-Methyl-2-phenyl-4-prop-2-enyloxazole (239).

Amide 256 (2.00 g, 9.205 mmol) was dissolved in TFA (16 mL) and TFAA (8 mL) was added. The mixture was heated at 35-40 °C for 16 h. The mixture was allowed to cool and the solvents removed under reduced pressure. The residue was diluted with saturated NaHCO₃ (50 mL) and solid NaHCO₃ was added to neutralize the mixture. It then was extracted with ethyl acetate (3 × 60 mL). The combined organic extracts were washed with brine, dried over MgSO₄, filtered and the solvent removed. Purification by flash chromatography on silica gel eluting with hexanes:ethyl acetate (15:1) gave oxazole 239 (1.75 g, 95%): ¹H NMR (CDCl₃, 400 MHz) δ 7.98 (d, *J* = 7.8 Hz, 2 H), 7.43-7.35 (m, 3 H), 6.03-5.93 (m, 1 H), 5.13 (dq*, J =* 16.9 and 1.7 Hz, 1 H), 5.09 (dq, *J* = 10.0 and 1.5 Hz, 1 H), 3.29 (d, *J* = 6.3 Hz, 2 H), 2.32 (s, 3 H); CIMS *m*/*z* 200 (M + H)⁺; IR 3070, 2924, 1638, 1450 cm⁻¹.

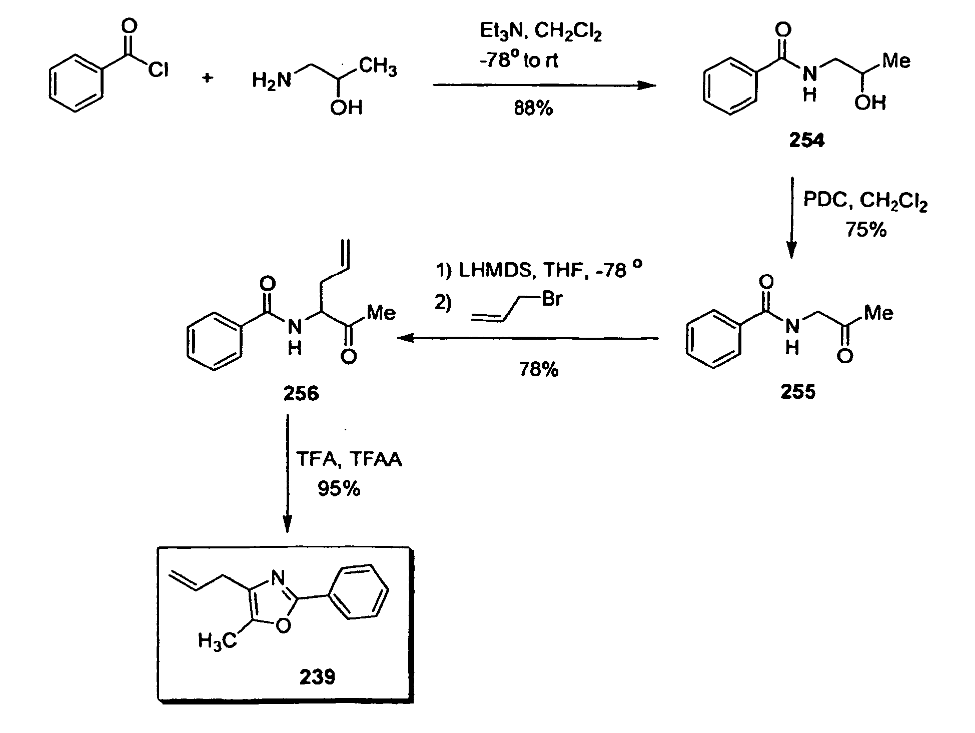

Amide (256) was prepared in the following manner.

### (a) N-(1-Acetylbut-3-enyl)benzamide (256).

Amide 255 (2.098 g, 11.839 mmol) was dissolved in THF (120 mL) and cooled to -78 °C under nitrogen. A 1.0 M solution of LHMDS in THF (11.9 mL, 11.9 mmol) was added and the mixture stirred for 40 min. A solution of allyl bromide (1.33 mL, 15.39 mmol) in THF (10 mL) was added. The mixture was allowed to warm to room temperature and stirred overnight. The mixture was diluted with brine and the phases were separated. The aqueous phase was extracted with ethyl acetate (3 × 50 mL) and the combined organic extracts were dried over MgSO₄, filtered, and the solvent removed. Purification by flash chromatography on silica gel eluting with hexanes:ethyl acetate (2:1 to 1:1) gave amide 256 (2.02 g, 78%): ¹H NMR (CDCl₃, 400 MHz) δ 7.79 (d, *J=* 7.1 Hz, 2 H), 7.51-7.41 (m, 3 H), 6.95 (bd, *J =* 5.4 Hz, 1 H), 5.74-5.64 (m, 1 H), 5.17-5.12 (m, 2 H), 4.88 (dt, *J*= 6.8 and 5.4 Hz, 1 H), 2.85-2.78 (m, 1 H), 2.61-2.54 (m, I H), 2.28 (s, 3 H); CIMS *mlz* 218 (M + H)⁺; IR 3263, 3081,1719,1632,1556 cm⁻¹. Anal. calcd for C₁₃H₁₅NO₂: C, 71.87; H, 6.96; N, 6.45. Found: C, 71.91; H, 7.03; N, 6.52.

### (b) Amide (255).

According to the method of Ellinger, L. P.; Goldberg, A. A. *J. Chem. Soc.* **1949**, 263. Alcohol 254 (3.0 g, 16.7 mmol) was dissolved in CH₂Cl₂ (60 mL) and PDC (9.42 g, 25.0 mmol) was added. The mixture was stirred at room temperature for 24 and more PDC (9.42 g, 25.0 mmol) was added. Stirring was continued for 24 h. The mixture was diluted with ethyl acetate and filtered through a celite pad. The residue was then passed through a shoroom temperature silica gel column eluting with ethyl acetate. The solvent was removed and the residue was purified by flash chromatography on silica gel eluting with hexanes:ethyl acetate (1:1) containing MeOH (0 to 4%). This purification afforded amide 255 as a white solid (2.21 g, 75%): ¹H NMR (CDCl₃, 400 MHz) δ 7.76 (dd, *J* = 7.0 and 1.5 Hz, 2 H), 7.48-7.36 (m, 3 H), 6.96 (bs, 1 H), 4.30 (d, *J* = 4.6 Hz, 2 H), 2.21 (s, 3 H); CIMS *m*/*z* 178 (M + H)⁺.

(b-1) An alternative route to benzamidoacetone 255 is shown below. This route eliminates the PDC oxidation that is tedious to work up. This method does not require chromatographic purification and affords amide 6 in 63% for the two steps.

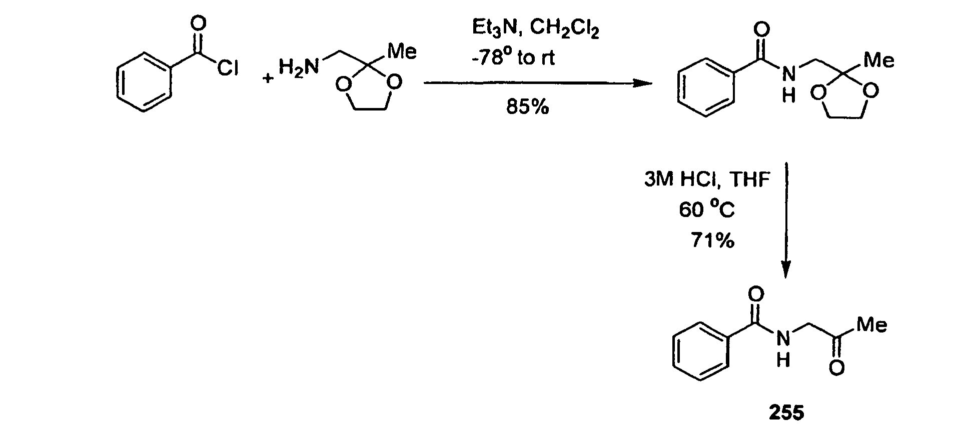

### (c) N-(2-Hydroxypropyl)benzamide (254).

The title compound was prepared according to the method of Arai, K.; Tamura, S.; Masumizu, T.; Kawai, K.-I.; Nakajima, S.; Ueda, A. *Can. J. Chem.* **1990**;*68*:903-907. A solution of DL-1-amino-2-propanol (3.4 mL, 43.2 mmol) and triethylamine (16.4 mL, 117.9 mmol) in CH₂Cl₂ (60 mL) under nitrogen was cooled at -78 °C. Benzoyl chloride (4.6 mL, 39.3 mmol) was added dropwise. The mixture was allowed to warm slowly and stirred at room temperature overnight. It was then diluted with CH₂Cl₂ (100 mL) and washed with cold 5% HCl and brine. The phases were separated and the aqueous phase was extracted with CH₂Cl₂ (2 × 50 mL). The combined organic extracts were dried over MgSO₄, filtered, and the solvent removed. The residue was dried under vacuum to give amide 254 as a pale yellow solid (6.21 g, 88%): ¹H NMR (CDCl₃, 400 MHz) δ 7.75 (d, *J* = 6.8 Hz, 2 H), 7.49-7.39 (m, 3 H), 6.57 (bs, 1 H), 4.00 (m, 1 H), 3.67-3.61 (m, 1 H), 3.31-3.24 (m, 1 H), 1.22 (d, *J*= 6.3 Hz, 3 H).

### Example 11

### 4-{4-[3-(5-Methyl-2-phenyl-oxazol-4-yl)-propyl]-phenyl}-2-pyrrol-1-yl-butyric acid (312).

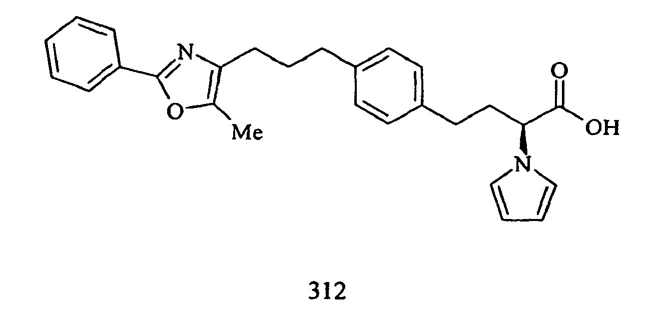

4-{4-[3-(5-Methyl-2-phenyl-oxazol-4-yl)-propyl]-phenyl}-2-pyrrol-1-yl-butyric acid methyl ester was hydrolyzed as in general procedure A to give carboxylic acid 312 in 90% yield. (δ) NMR (CHCl₃) 7.86 (2H, m); 7.33 (3H, m); 7.03 (2H, d, J=8.1 Hz); 6.97 (2H, d, J=8.8Hz), 6.68 (2H, m); 6.14 (2H, m); 4.46 (1H, m); 2.56 (3H, m); 2.44 (5H, m); 2.22 (3H, s); 1.88 (2H, m). CIMS *mlz* 429.3 (M)⁺.

4-{4-[3-(5-Methyl-2-phenyl-oxazol-4-yl)-propyl]-phenyl}-2-pyrrol-1-yl-butyric acid methyl ester was prepared in the following manner.

### (a) 4-{4-[3-(5-Methyl-2-phenyl-oxazol-4-yl)-propyl]-phenyl}-2-pyrrol-1-yl-butyric acid methyl ester (311).

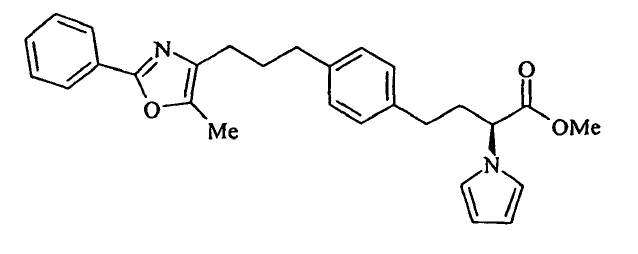

The title compound was prepared as in the procedure outlined for the preparation of esters 120 a-d in 38% yield. 2-Pyrrol-1-yl-4-(4-trifluoromethanesulfonyl-phenyl)-butyric acid methyl ester was used as the triflate component. (δ) NMR (CHCl₃) 8.00 (2H, m); 7.47 (3H, m); 7.10 (2H, d, J=7.6Hz); 7.62 (2H, d, J=7.6Hz); 6.74 (2H, s); 6.21 (2H, s); 4.50 (1H, m); 3.69 (3H, s); 2.58 (6H, m); 2.37 (2H0; 2.31 (3H, s); 1.99 (2H, m). CIMS *m*/*z* 44.3 (M)⁺.

### Example 12

### 2-Pyrrol-1-yl-4-(4-trifluoromethanesulfonyl-phenyl)-butyric acid methyl ester (314).

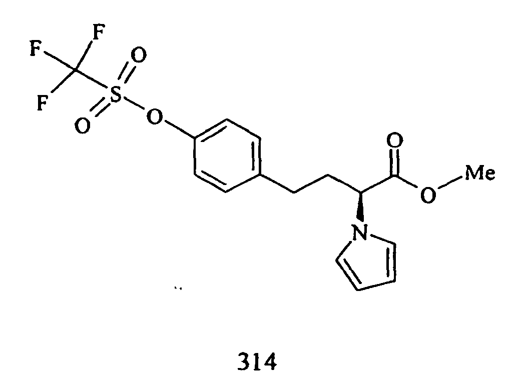

The title compound was prepared as described in the synthesis of (*S*)-2-Pyrrol-1-yl-3-[(4-trifluoromethanesulfonyloxy)phenyl]-propionic acid methyl ester (119) from homo-tyrosine (CAS 141899-12-9) via esterification and pyrrole condensation as described in the preparation of pyrrolotyrosine methyl ester (1). (δ) NMR (CHCl₃) 7.18 (4H, s); 6.71 (2H, s); 6.21 (2H, s); 4.51 (1H, m); 3.70 (3H, s); 2.45 (4H, s). CIMS *m*/*z* 391.9 (M)⁺.

### Example 13

The following illustrates representative pharmaceutical dosage forms, containing a compound of Formula I (Compound 4f), for therapeutic or prophylactic use in humans.

| (i) Tablet | mg/tablet |
|---|---|
| Invention Compound | 25.0 |
| Lactose | 50.0 |
| Corn Starch (for mix) | 10.0 |
| Corn Starch (paste) | 10.0 |
| Magnesium Stearate (1%) | 3.0 |
| | 300.0 |

The biphenylsulfonamide, lactose, and corn starch (for mix) are blended to uniformity. The corn starch (for paste) is suspended in 200 mL of water and heated with stirring to form a paste. The paste is used to granulate the mixed powders. The wet granules are passed through a No. 8 hand screen and dried at 80°C. The dry granules are lubricated with the 1% magnesium stearate and pressed into a tablet. Such tablets can be administered to a human from one to four times a day for treatment of pathogenic bacterial infections.

| (ii) Tablet | mg/capsule |
|---|---|
| Invention Compound | 10.0 |
| Colloidal Silicon Dioxide | 1.5 |
| Lactose | 465.5 |
| Pregelatinized Starch | 120.0 |
| Magnesium Stearate (1%) | 3.0 |
| | 600.0 |

| (iii) Preparation for Oral Solution | Amount |
|---|---|
| 'Compound 4f' | 400 mg |
| Sorbitol Soluition (70 % N.F.) | 40 mL |
| Sodium Benzoate | 20 mg |
| Saccharin | 5 mg |
| Cherry Flavor | 20 mg |
| Distilled Water q.s. | 100 mL |

The sorbitol solution is added to 40 mL of distilled water, and the biphenylsulfonamide is dissolved therein. The saccharin, sodium benzoate, flavor, and dye are added and dissolved. The volume is adjusted to 100 mL with distilled water. Each milliliter of syrup contains 4 mg of invention compound.

### (iv) Parenteral Solution

In a solution of 700 mL of propylene glycol and 200 mL of water for injection is suspended 20 g of 2-Amino-4-cyclopropyl-7-fluoro-5-methyl-6-[3-(1-methylamino-ethyl)-pyrrolidin-1-yl]-pyrido[1,2-*c*]pyrimidine-1,3-dione (Compound 4f). After suspension is complete, the pH is adjusted to 6.5 with 1 N hydrochloric acid, and the volume is made up to 1000 mL with water for injection. The Formulation is sterilized, filled into 5.0 mL ampoules each containing 2.0 mL, and sealed under nitrogen.

| (v) Injection 1 (1 mg/mL) | Amount |
|---|---|
| Invention Compound | 1.0 |
| Dibasic Sodium Phosphate | 12.0 |
| Monobasic Sodium Phosphate | 0.7 |
| Sodium Chloride | 4.5 |
| N Sodium hydroxide solution (pH adjustment to 7.0-7.5) | q.s. |
| Water for injection | q.s. ad 1 mL |

| (vi) Injection 2 (10 mg/mL) | Amount |
|---|---|
| Invention Compound | 10.0 |
| Dibasic Sodium Phosphate | 1.1 |
| Monobasic Sodium Phosphate | 0.3 |
| Polyethylene glyco 400 | 200.0 |
| N hydrochloric acid solution (pH adjustment to 7.0-7.5) | q.s. |
| Water for injection | q.s. ad 1 mL |

| (vii) Injection 2 (10 mg/mL) | Amount |
|---|---|
| Invention Compound | 20.0 |
| Oleic Acid | 10.0 |
| Trichloromonofluoromethane | 5,000.0 |
| Dichlorodifluoromethane | 10,000.0 |
| Dichlorotetrafluoroethane | 5,000.0. |

## Claims

1. A compound of formula (I) 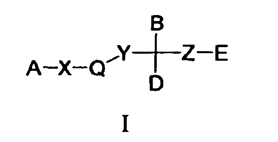 or a pharmaceutically acceptable salt thereof, wherein:
A is 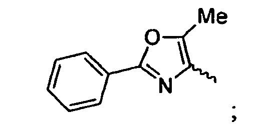 X is 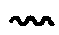 CH₂-CH₂-O 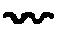 or -CH₂CH₂CH₂- ;
Q is 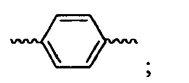 Y is CH₂;
Z is absent;
B is H;
D is 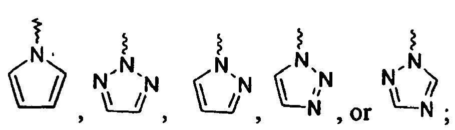 and
E is CO₂H.

2. A compound according to claim 1 selected from:

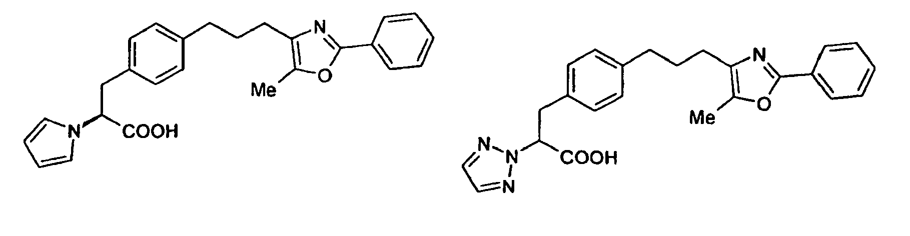

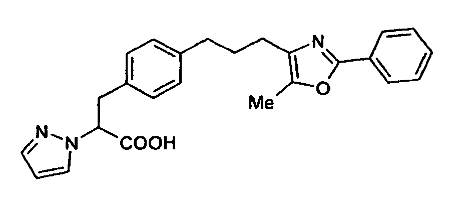

3. A pharmaceutical composition comprising a compound of Claim 1 admixed with a carrier, diluent, or excipient.

4. A compound of claim 1 for use as a medicament.

5. The use of a compound of claim 1 in the preparation of a medicament for treating, preventing or controlling non-insulin dependent diabetes mellitus, obesity, hyperglycemia, hyperlipidemia, hypercholesteremia, atherosclerosis, hypertriglyceridemia, hyperinsulinemia, or insulin resistance syndrome in a mammal.

6. The use of a compound of formula I in the preparation of a medicament for treating, preventing or controlling a condition ameliorated by modulating PPAR activity, lowering blood glucose, or modulating fat cell differentiation in a mammal.

## Patentansprüche

1. Verbindung der Formel (I) 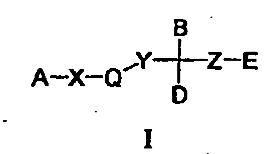 oder ein pharmazeutisch akzeptables Salz derselben, worin:
A für 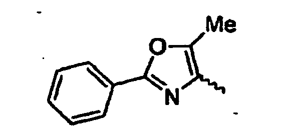 steht;
X für
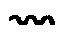 CH₂-CH₂-O 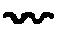 oder -CH₂CH₂CH₂- steht;
Q für 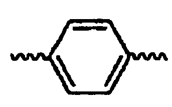 steht;
Y für CH₂ steht;
Z nicht vorhanden ist;
B für H steht;
D für 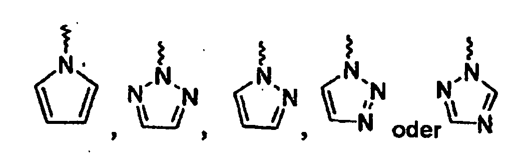 steht; und
E für CO₂H steht.

2. Verbindung nach Anspruch 1, die ausgewählt ist aus:

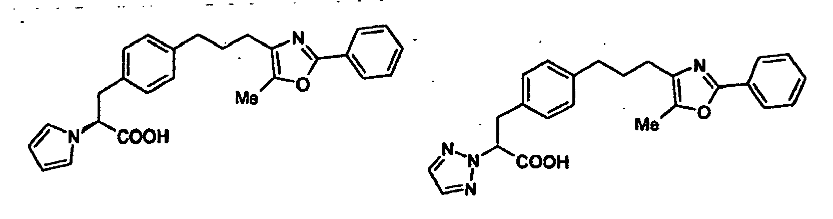

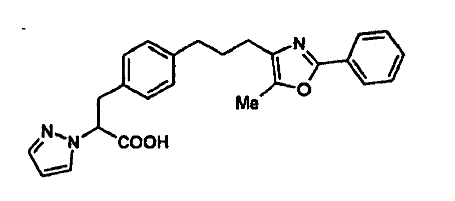

3. Pharmazeutische Zusammensetzung, die eine Verbindung nach Anspruch 1 im Gemisch mit einem Träger, Verdünnungsmittel oder Streckmittel umfasst.

4. Verbindung nach Anspruch 1 zur Verwendung als Medikament.

5. Verwendung einer Verbindung nach Anspruch 1 bei der Herstellung eines Medikaments zur Behandlung, Prävention oder Kontrolle von nicht-insulinabhängigem Diabetes mellitus, Fettsucht, Hyperglykämie, Hyperlipidämie, Hypercholesterinämie, Atherosklerose, Hypertriglyceridämie, Hyperinsulinämie oder Insulinresistenzsyndrom bei einem Säuger.

6. Verwendung einer Verbindung der Formel I bei der Herstellung eines Medikaments zur Behandlung, Prävention oder Kontrolle eines Zustands, der durch Modulation der PPAR-Aktivität, Senkung der Blutglucose oder Modulation der Fettzellendifferenzierung verbessert wird, bei einem Säuger.

## Revendications

1. Composé de formule (I) 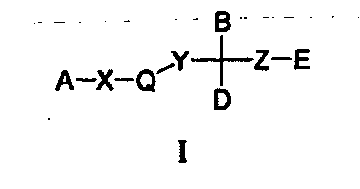 ou sel pharmaceutiquement acceptable d'un tel composé, dans lequel :
A représente 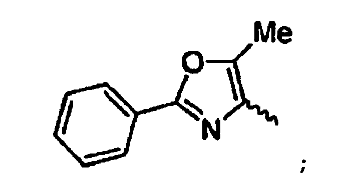
X représente 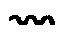 CH₂-CH₂-O 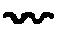 ou -CH₂CH₂CH₂- ;
Q représente 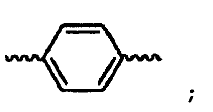
Y représente CH₂ ;
Z est absent ;
B représente H ;
D représente 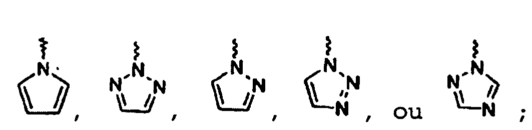 et
E représente CO₂H.

2. Composé selon la revendication 1, sélectionné parmi :

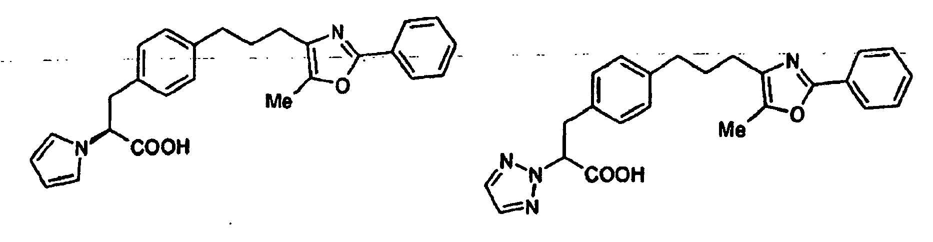

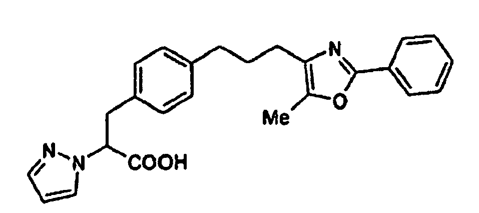

3. Composition pharmaceutique comprenant un composé selon la revendication 1, en mélange avec un support, un diluant ou un excipient.

4. Composé selon la revendication 1, pour une utilisation en tant que médicament.

5. Utilisation d'un composé selon la revendication 1, pour la préparation d'un médicament destiné à traiter, prévenir ou contrôler le diabète non insulino-dépendant, l'obésité, l'hyperglycémie, l'hyperlipidémie, l'hypercholestérolémie, l'athérosclérose, l'hypertriglycéridémie, l'hyperinsulinémie, ou le syndrome de résistance à l'insuline, chez un mammifère.

6. Utilisation d'un composé de formule I pour la préparation d'un médicament destiné à traiter, prévenir ou contrôler un état amélioré par une modulation de l'activité du PPAR, par un abaissement du glucose sanguin, ou par une modulation de la différenciation des cellules graisseuses, chez un mammifère.
